(19) Europäisches Patentamt
European Patent Office
Office européen des brevets

(11) EP 4 245 844 A1

(12) EUROPEAN PATENT APPLICATION
published in accordance with Art. 153(4) EPC

(43) Date of publication:
20.09.2023 Bulletin 2023/38

(21) Application number: 21891881.1

(22) Date of filing: 10.11.2021

(51) International Patent Classification (IPC):
*C12N 1/21* *(2006.01)* *C12N 15/52* *(2006.01)*
*C12P 7/44* *(2006.01)*

(52) Cooperative Patent Classification (CPC):
C12N 15/52; C12N 15/74; C12P 7/44

(86) International application number:
PCT/JP2021/041257

(87) International publication number:
WO 2022/102635 (19.05.2022 Gazette 2022/20)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 11.11.2020 JP 2020187848

(71) Applicant: Toray Industries, Inc.
Tokyo 103-8666 (JP)

(72) Inventors:
- ISOBE, Kyohei
Kamakura-shi, Kanagawa 248-8555 (JP)
- KAWAMURA, Kenji
Kamakura-shi, Kanagawa 248-8555 (JP)
- YAMADA, Katsushige
Kamakura-shi, Kanagawa 248-8555 (JP)

(74) Representative: Kador & Partner Part mbB
Corneliusstraße 15
80469 München (DE)

Remarks:
The complete document including Reference Table(s) and the Sequence Listing(s) can be downloaded from the EPO website

# (54) GENETICALLY MODIFIED MICROORGANISM FOR PRODUCING 3-HYDROXYADIPIC ACID AND/OR ?-HYDROMUCONIC ACID, AND METHOD FOR PRODUCING CHEMICAL PRODUCT

(57) Disclosed are a genetically modified microorganism that can produce 3-hydroxyadipic acid and/or α-hydromuconic acid in high yields, and the genetically modified microorganism. The genetically modified microorganism is a microorganism having an ability to produce 3-hydroxyadipic acid and/or α-hydromuconic acid, in which the reaction to generate acetyl-CoA from pyruvic acid is enhanced and function of pyruvate kinase and/or phosphotransferase system is reduced.



Processed by Luminess, 75001 PARIS (FR)

**Description**

TECHNICAL FIELD

The present invention relates to a genetically modified microorganism that produces 3-hydroxyadipic acid and/or α-hydromuconic acid in high yields, and a method of producing 3-hydroxyadipic acid and/or α-hydromuconic acid using the genetically modified microorganism.

BACKGROUND ART

3-Hydroxyadipic acid (IUPAC name: 3-hydroxyhexanedioic acid) and α-hydromuconic acid (IUPAC name: (E)-hex-2-enedioic acid) are dicarboxylic acids containing six carbon atoms. These dicarboxylic acids can be used as raw materials for the production of polyesters by polymerization with polyols or as raw materials for the production of polyamides by polymerization with polyamines. Additionally, compounds obtained by adding ammonia to the end of these dicarboxylic acids and converting the resultants to lactams can also be used as raw materials for polyamides.

The following documents are known concerning the production of 3-hydroxyadipic acid or α-hydromuconic acid by using microorganisms.

Patent Document 1, as a document concerning the production of C6 dicarboxylic acid using microorganisms, describes a method of producing 3-hydroxyadipic acid, α-hydromuconic acid, and/or adipic acid by using polypeptide showing excellent catalytic activity in the reduction reaction from 3-oxoadipyl-CoA to 3-hydroxyadipyl-CoA. It is described in the document that the biosynthesis pathway for these substances proceeds through an enzymatic reaction that reduces 3-oxoadipyl-CoA to 3-hydroxyadipyl-CoA. However, all the genes that are modified in Patent Document 1 are limited to reactions on the above-described biosynthesis pathway in cells, and there is no description on the reduction or enhancement of the enzyme activity in the metabolic pathway upstream of said reactions.

Patent Document 2 describes a method of producing 1,3-butadiene by using a microorganism having modified metabolic pathway. In this document, 3-hydroxyadipic acid (3-hydroxyadipate) is described as a metabolic intermediate in the metabolic pathway for biosynthesis of 1,3-butadiene from acetyl-CoA and succinyl-CoA.

Patent Document 3 describes a method of producing muconic acid by using a microorganism having modified metabolic pathway. In this document, α-hydromuconic acid (2,3-dehydroadipate) is described as a metabolic intermediate in the metabolic pathway for biosynthesis of trans, trans-muconic acid from acetyl-CoA and succinyl-CoA.

It is described that all the biosynthesis pathways described in Patent Documents 2 and 3 proceed through an enzymatic reaction that reduces 3-oxoadipyl-CoA to 3-hydroxyadipyl-CoA.

Patent Documents 4 and 5 describe methods of producing adipic acid and hexamethylenediamine (HMDA) by using non-naturally occurring microorganisms. In these documents, biosynthesis pathways for these substances are described, which are common in that the biosynthesis pathways include the reaction to synthesize 3-oxoadipyl-CoA from acetyl-CoA and succinyl-CoA. However, other biosynthesis pathways are described up to the generation of 3-hydroxyadipic acid or α-hydromuconic acid from 3-oxoadipyl-CoA.

Patent Document 4 describes, for the production of HMDA, pyruvate kinase as additional gene deletion to improve the formation of HMDA in conjugation with the proliferation, and a phosphotransferase system as additional gene deletion to improve the yield. On the other hand, for the production of adipic acid, there is description on the phosphotransferase system as additional gene deletion to improve the formation of adipic acid in conjugation with the proliferation, but no description on pyruvate kinase. For both the production of HMDA and adipic acid, there is no description on the enhancement of the reaction to generate acetyl-CoA from pyruvic acid.

Patent Document 5 describes production of adipic acid using a non-naturally occurring microorganism having phosphoketolase, in which the activity of phosphotransferase system and pyruvate kinase is attenuated or removed. It also describes genetic modification comprising enhancement of the reaction to generate acetyl-CoA from pyruvic acid for the production of NADH in the presence of phosphoketolase.

Patent Document 6 discloses methods of improving microorganisms based on in silico analysis, in which deletion of pykF, pykA, and ptsG which are genes encoding pyruvate kinase and phosphotransferase system of *Escherichia coli,* and culturing under anaerobic conditions result in increased production of succinic acid.

Non-Patent Document 1 discloses that deletion of pdhR, which is a gene encoding a transcriptional repressor for the pyruvate dehydrogenase complex of *Escherichia coli,* results in increased production of 2-oxoglutaric acid.

PRIOR ART DOCUMENTS

PATENT DOCUMENTS

Patent Document 1: WO 2019/107516
Patent Document 2: JP 2013-535203 A
Patent Document 3: US 2011/0124911 A1
Patent Document 4: JP 2015-146810 A
Patent Document 5: US 2017/0298363 A1
Patent Document 6: JP 2008-527991 A

NON-PATENT DOCUMENT

Non-patent Document 1: J Biosci Bioeng. 2017 Apr; 123(4): 437-443.

SUMMARY OF THE INVENTION

PROBLEMS TO BE SOLVED BY THE INVENTION

Patent Document 1 discloses enhanced expression of enzyme genes that play roles in improving the productivity for 3-hydroxyadipic acid or α-hydromuconic acid. However, all the enzyme genes with enhanced expression are limited to reactions downstream of acetyl-CoA and succinyl-CoA in the biosynthesis pathway, and enhancement of the enzyme activity in the metabolic pathway upstream of them is not described. Patent Documents 2 and 3 describe the metabolic pathways that can produce 3-hydroxyadipic acid and α-hydromuconic acid in the microorganisms. However, there is no description about stopping the metabolism at 3-hydroxyadipic acid or α-hydromuconic acid to secrete 3-hydroxyadipic acid and α-hydromuconic acid into culture medium. Moreover, it has not been examined whether the use of the microorganisms into which the enzyme gene that catalyzes the reaction to reduce 3-oxoadipyl-CoA to 3-hydroxyadipyl-CoA is introduced as described in Patent Documents 2 to 5 actually enables production of 3-hydroxyadipic acid or α-hydromuconic acid. Patent Documents 6 and non-Patent Document 1 do not describe anything about 3-hydroxyadipic acid or α-hydromuconic acid.
In view of this, an object of the present invention is to provide a genetically modified microorganism to produce 3-hydroxyadipic acid and/or α-hydromuconic acid in high yields, based on a genetically modified microorganism in which a gene encoding 3-oxoadipyl-CoA reductase is introduced, or the expression of the gene is enhanced to exhibit enhanced activity of the enzyme, by further modifying upstream metabolic pathway; and to provide a method of producing substances using the modified microorganism.

MEANS FOR SOLVING THE PROBLEMS

The present inventor has intensively studied in order to achieve the object described above and consequently found that a genetically modified microorganism having an ability to produce 3-hydroxyadipic acid and/or α-hydromuconic acid, in which the reaction to generate acetyl-CoA from pyruvic acid is enhanced, and the function of pyruvate kinase and/or phosphotransferase system is reduced, enables production of 3-hydroxyadipic acid and/or α-hydromuconic acid in high yields, and exhibits excellent ability to produce 3-hydroxyadipic acid and/or α-hydromuconic acid, thereby completing the present invention.
That is, the present invention provides the following:

(1) A genetically modified microorganism having an ability to produce 3-hydroxyadipic acid and/or α-hydromuconic acid, wherein the reaction to generate acetyl-CoA from pyruvic acid is enhanced and function of pyruvate kinase and/or phosphotransferase system is reduced;
(2) The genetically modified microorganism of (1), wherein the enhancement of the reaction to generate acetyl-CoA from pyruvic acid is an enhancement of the reaction catalyzed by pyruvate dehydrogenase complex and/or an enhancement of the reaction catalyzed by pyruvate formate-lyase;
(3) The genetically modified microorganism of (2), wherein the enhancement of the reaction catalyzed by the pyruvate dehydrogenase complex is an enhancement by increased expression of the pyruvate dehydrogenase complex and/or increased activity of the pyruvate dehydrogenase complex;
(4) The genetically modified microorganism of (3), wherein the increased expression of the pyruvate dehydrogenase complex is achieved by reducing the function of transcriptional repressor of the pyruvate dehydrogenase complex;
(5) The genetically modified microorganism of (3), wherein the increased activity of the pyruvate dehydrogenase complex is achieved by reducing the sensitivity of the pyruvate dehydrogenase complex to NADH;
(6) The genetically modified microorganism of (2), wherein the enhancement of the reaction catalyzed by pyruvate formate-lyase is achieved by enhancement by increased expression of pyruvate formate-lyase;
(7) The genetically modified microorganism of any one of (1) to (6), wherein the reaction that reduces 3-oxoadipyl-CoA to generate 3-hydroxyadipyl-CoA is further enhanced;

(8) The genetically modified microorganism of any one of (1) to (7), wherein phosphoenolpyruvate carboxykinase reaction is further enhanced;

(9) The genetically modified microorganism of any one of (1) to (8), wherein the microorganism does not undergo glucose metabolism via the phosphoketolase pathway;

(10) A method of producing 3-hydroxyadipic acid and/or α-hydromuconic acid, comprising the step of culturing a genetically modified microorganism of any one of (1) to (9);

(11) A method of producing a genetically modified microorganism having an ability to produce 3-hydroxyadipic acid and/or α-hydromuconic acid, comprising a step of enhancing or reducing functions inherent in a microorganism by genetic modification,
wherein the step comprises:

a step (a) of enhancing the reaction to generate acetyl-CoA from pyruvic acid, and
a step (b) of reducing the enzyme function of pyruvate kinase and/or phosphotransferase system.

(12) The method according to (11), further comprising a step (c) of enhancing the reaction that reduces 3-oxoadipyl-CoA to generate 3-hydroxyadipyl-CoA;

(13) The method according to (11) or (12), further comprising a step (d) of enhancing the phosphoenolpyruvate carboxykinase reaction.

EFFECT OF THE INVENTION

A microorganism having an ability to produce 3-hydroxyadipic acid and/or α-hydromuconic acid, with genetic modification to enhance the reaction to generate acetyl-CoA from pyruvic acid and reduce the function of pyruvate kinase and/or phosphotransferase system, can produce 3-hydroxyadipic acid and/or α-hydromuconic acid in higher yields than the parent microorganism strain without modification of the gene.

DETAILED DESCRIPTION OF THE INVENTION

It has been found in the present invention that the microorganism having an ability to produce 3-hydroxyadipic acid and/or α-hydromuconic acid, in which the reaction to generate acetyl-CoA from pyruvic acid is enhanced and the function of pyruvate kinase and/or phosphotransferase system is reduced, enables production of 3-hydroxyadipic acid and/or α-hydromuconic acid in high yields.

Hereinafter, 3-hydroxyadipic acid may be abbreviated as 3HA, and α-hydromuconic acid may be abbreviated as HMA. In addition, 3-oxoadipyl-CoA may be abbreviated as 3OA-CoA, 3-hydroxyadipyl-CoA may be abbreviated as 3HA-CoA, and 2,3-dehydroadipyl-CoA may be abbreviated as HMA-CoA. In addition, phosphoenolpyruvate may be abbreviated as PEP. In addition, the enzyme that catalyzes the reaction where 3-oxoadipyl-CoA is reduced to generate 3-hydroxyadipyl-CoA may be referred to as "3-oxoadipyl-CoA reductase." In addition, the complex of the proteins encoded by the aceE, aceF and lpd genes in the presence of a single promoter may be referred to as pyruvate dehydrogenase complex and abbreviated as PDHc. In addition, the aceE, aceF and lpd genes may be collectively referred to as PDHc gene cluster. In addition, pyruvate formate-lyase and the pyruvate formate-lyase activating enzyme may be collectively referred to as pyruvate formate-lyase, and abbreviated as PFL or Pfl. In addition, pyruvate kinase may be abbreviated as Pyk, and the phosphotransferase system may be abbreviated as PTS. In addition, a nucleic acid encoding a functional polypeptide may be referred to as a gene.

The genetically modified microorganism of the present invention can biosynthesize 3-hydroxyadipic acid and/or α-hydromuconic acid via acetyl-CoA and succinyl-CoA as intermediates, as shown in the metabolic pathway described below. The metabolic pathway from glucose up to acetyl-CoA is well-known as glycolytic pathway, and the metabolic pathway up to succinyl-CoA as TCA cycle.

[Chem 1]

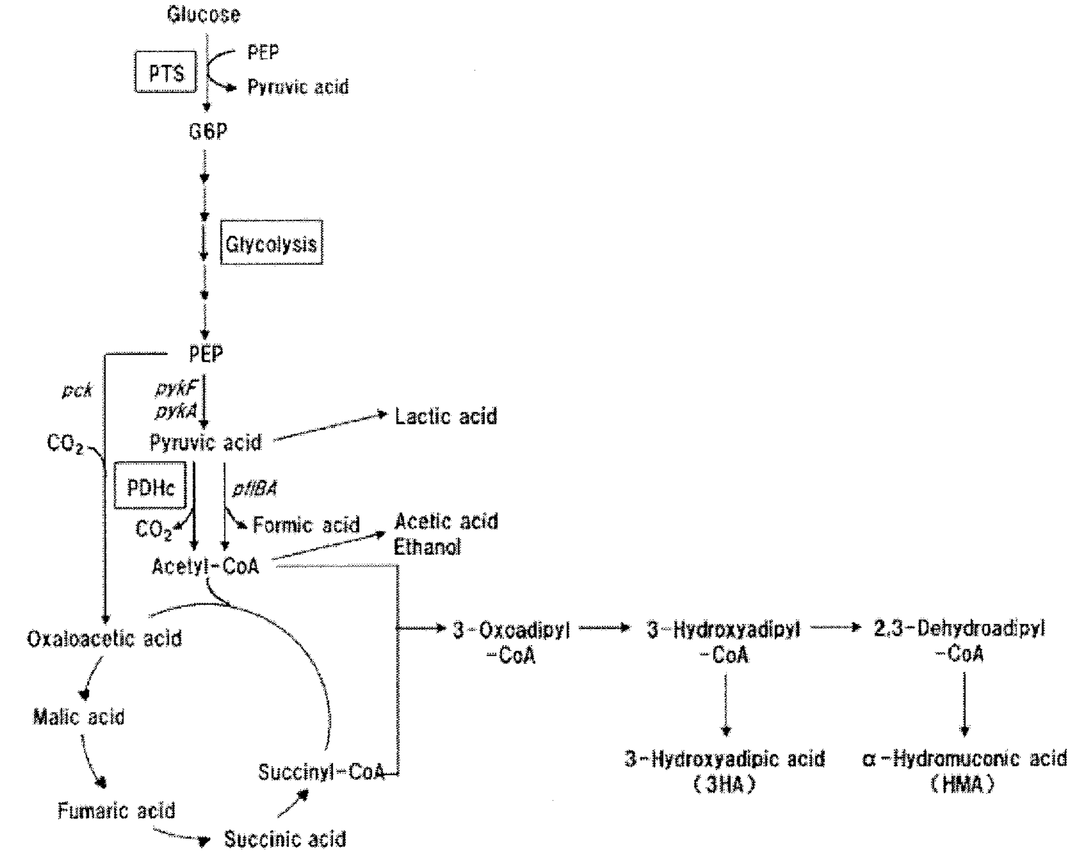


The metabolic pathways to produce 3-hydroxyadipic acid and/or α-hydromuconic acid from acetyl-CoA obtained in the glycolytic pathway and succinyl-CoA obtained in the TCA cycle are shown below, with the metabolites represented by the chemical formulae. In this scheme, the reaction A represents a reaction to generate 3-oxoadipyl-CoA from acetyl-CoA and succinyl-CoA. The reaction B represents a reaction that reduce 3-oxoadipyl-CoA to generate 3-hydroxyadipyl-CoA. The reaction C represents a reaction to generate 2,3-dehydroadipyl-CoA from 3-hydroxyadipyl-CoA. The reaction D represents a reaction to generate 3-hydroxyadipic acid from 3-hydroxyadipyl-CoA. The reaction E represents a reaction to generate α-hydromuconic acid from 2,3-dehydroadipyl-CoA. The enzymes that catalyze the reactions in the following metabolic pathway, and the method of creating a microorganism having an ability to produce 3-hydroxyadipic acid and/or α-hydromuconic acid using the following metabolic pathway are described in detail in WO2019/107516.

[Chem 2]

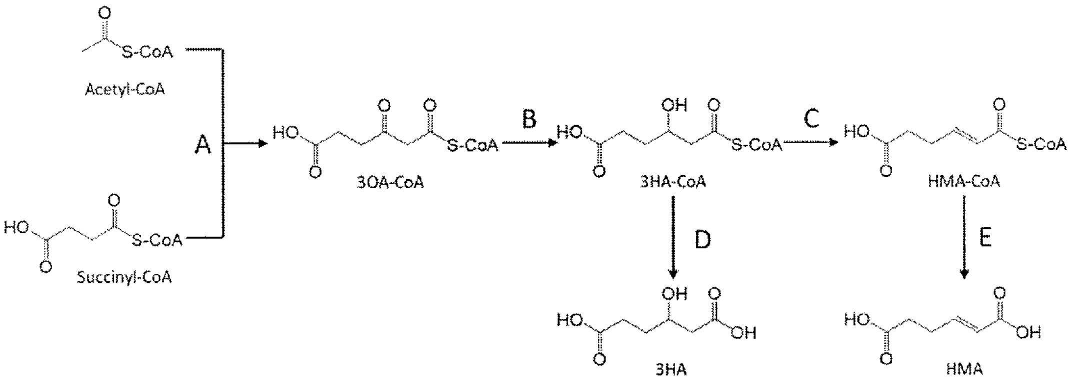

Acetyl-CoA
Succinyl-CoA
3OA-CoA
3HA-CoA
HMA-CoA
3HA
HMA

One method to enhance the reaction to generate acetyl-CoA from pyruvic acid in the present invention include enhancing the reaction catalyzed by the pyruvate dehydrogenase complex (PDHc). The PDHc to be enhanced is not particularly limited, provided that it has a catalytic activity for the production of acetyl-CoA from pyruvic acid. For example,

in bacteria such as the genus *Escherichia* and the genus *Serratia*, the PDHc is composed of pyruvate dehydrogenase (E1, EC1.2.4.1), dihydrolipoyl transacetylase (E2, EC2.3.1.12), and dihydrolipoyl dehydrogenase (E3, EC1.8.1.4), which are encoded by aceE, aceF, and lpd genes, respectively. PDHc catalyzes as a whole the reactions that produce acetyl-CoA, $CO_2$, and NADH from pyruvic acid, coenzyme A, and $NAD^+$. The PDHc gene cluster form an operon, and the expression of the PDHc gene cluster is regulated by a PDHc transcriptional repressor (PdhR) encoded by the pdhR gene. The transcription of the PDHc gene cluster is repressed in the presence of PdhR, so that the expression of PDHc is decreased. On the other hand, the transcription of the PDHc gene cluster is not inhibited in the absence of PdhR, so that the expression of PDHc is increased.

Specific examples of PDHc include AceE (NCBI-Protein ID: NP_414656), AceF (NCBI-Protein ID: NP_414657), and Lpd (NCBI-Protein ID: NP_414658) derived from *Escherichia coli* str. K-12 substr. MG1655; AceE (SEQ ID NO: 1), AceF (SEQ ID NO: 2), and Lpd (SEQ ID NO: 3) derived from *Serratia grimesii* NBRC13537; and LpdA (NCBI-Protein ID: ABR75580) derived from *Klebsiella pneumoniae* subsp. pneumoniae MGH 78578. Whether or not the polypeptide encoded by a gene possessed by the microorganism used in the present invention is PDHc can be determined by performing BLAST search on the public database in NCBI (National Center for Biotechnology Information), KEGG (Kyoto Encyclopedia of Genes and Genomes), or the like.

Specific examples of the PDHc transcriptional repressor include PdhR (NCBI-Protein ID: NP_414655) derived from *Escherichia coli* str. K-12 substr. MG1655, and PdhR (SEQ ID NO: 4) derived from *Serratia grimesii* NBRC13537. Whether or not the polypeptide encoded by a gene possessed by the microorganism used in the present invention is a PDHc transcriptional repressor can be determined by performing BLAST search on the public database in NCBI (National Center for Biotechnology Information), KEGG (Kyoto Encyclopedia of Genes and Genomes), or the like.

Method of enhancing the reaction catalyzed by PDHe include, for example, increasing the expression of at least one or more enzymes that constitute PDHc. Methods of increasing the expression of at least one or more enzymes that constitute PDHc include, for example, introducing at least one or more genes that constitutes the PDHc gene cluster into host microorganisms from outside the microorganisms; increasing the copy numbers of the gene cluster; and modifying the promoter regions or the ribosome-binding sequences upstream of the coding regions of the gene cluster. These methods may be carried out individually or in combination. The increase in the expression of PDHc can also be achieved by decreasing the function of the PDHc transcriptional repressor.

Other methods of enhancing the reaction catalyzed by PDHc include, for example, enhancing the activity of PDHc. The catalytic activity of at least one or more enzymes constituting PDHc may be enhanced to enhance the activity of PDHc. Specific methods of enhancing the PDHc activity include, for example, reducing the sensitivity to NADH. Reduced sensitivity to NADH means, for example, that the Ki value of the enzyme for NADH is two or more times higher than the control. PDHc with reduced sensitivity for NADH is obtained by using a E354K and/or H322Y variant of Lpd derived from *Escherichia coli* str. K-12 (NCBI-Protein ID: NP_414658) described in Kim et al., J. Bacteriol. 190: 3851-3858 (2008), or using Lpd derived from *Klebsiella pneumoniae* (NCBI-Protein ID: ABR75580) or a part thereof, which is known to function under anaerobic environments. The enzyme gene with enhanced catalytic activity may be substituted for or coexist with the wild-type gene originally contained in the microorganism used in the production. Alternatively, the copy number of the enzyme gene may be increased, or the promoter region or ribosome-binding sequence upstream of the coding region of the enzyme gene may be modified. These methods may be carried out individually or in combination.

One method to enhance the reaction to generate acetyl-CoA from pyruvic acid in the present invention include enhancing the reaction catalyzed by pyruvate formate-lyase. The pyruvate formate-lyase to be enhanced is not particularly limited, provided that it has a catalytic activity for production of acetyl-CoA from pyruvic acid. For example, in bacteria such as *Escherichia* and *Serratia,* pyruvate formate-lyase (EC2.3.1.54) functions in the presence of pyruvate formate-lyase activating enzyme (EC1.97.1.4), which are encoded by pflB and pflA genes, respectively. Pyruvate formate-lyase catalyzes the reactions that produce acetyl-CoA and formic acid from pyruvic acid and coenzyme A. pflB and pflA form an operon.

Specific examples of pyruvate formate-lyase include PflB (NCBI-Protein ID: NP_415423) and PflA (NCBI-Protein ID: NP_415422) derived from *Escherichia coli* str. K-12 substr. MG1655, and PflB (SEQ ID NO: 5) and PflA (SEQ ID NO: 6) derived from *Serratia grimesii* NBRC13537. Whether or not the polypeptide encoded by a gene possessed by the microorganism used in the present invention is pyruvate formate-lyase can be determined by performing BLAST search on the public database in NCBI, KEGG, or the like.

The method of enhancing the reaction catalyzed by pyruvate formate-lyase may be, for example, enhancing the catalytic activity of pyruvate formate-lyase itself, or increasing the expression of pyruvate formate-lyase, and preferably is increasing the expression of pyruvate formate-lyase. Methods of increasing the expression of pyruvate formate-lyase include, for example, introducing the pyruvate formate-lyase gene into host microorganisms from outside the microorganisms; increasing the copy number of the gene; and modifying the promoter region or the ribosome binding sequence upstream of the coding region of the gene. These methods may be carried out individually or in combination.

Incidentally, US 2017/0298363 A1 describes production of adipic acid by using a non-naturally occurring microorganism, in which the metabolism of the microorganism having the phosphoketolase pathway is modified to increase

the energy efficiency. As described in the document, the glucose metabolism through the glycolytic pathway generates NADH, but the glucose metabolism through the phosphoketolase pathway does not generate NADH. Thus, in the case of production of reduced compounds using a microorganism having the phosphoketolase pathway, the expression or activity of PDHc or pyruvate formate-lyase and an NAD(P)H-generating formate dehydrogenase is enhanced in order to improve the shortage of NADH in the metabolism of the microorganism. Here, adipic acid is a more reduced compound than 3-hydroxyadipic acid and $\alpha$-hydromuconic acid, and thus it is thought that those skilled in the art will expect that enhancing the PDHc and/or pyruvate formate-lyase reaction(s) in production of 3-hydroxyadipic acid and/or $\alpha$-hydromuconic acid leads to oxidation-reduction imbalance, and the yield of the compound is decreased. However, the present invention gives an unexpected effect that culturing of a genetically modified microorganism having an ability to produce 3-hydroxyadipic acid and/or $\alpha$-hydromuconic acid, in which the reaction to generate acetyl-CoA from pyruvic acid is enhanced, results in improved production of 3-hydroxyadipic acid and/or $\alpha$-hydromuconic acid.

In the present invention, reducing the function of pyruvate kinase and/or phosphotransferase system means reducing the activity of the enzymes. The method of reducing the functions is not particularly limited, and the reduction can be achieved, for example, by downregulating the genes encoding the enzymes through gene mutation treatment such as by gene mutation agent or ultraviolet irradiation, treatment to delete a portion or the whole of the nucleotide sequence such as by site-specific mutagenesis, introduction of frameshift mutation into the nucleotide sequence, introduction of stop codon into the nucleotide sequence, or the like. The reduction can also be achieved by removing the entire or a part of the nucleotide sequence or substituting it with other nucleotide sequence using gene recombination technology. Among them, preferred is a method in which a part or the whole of the nucleotide sequence is removed.

Pyruvate kinases (Pyk) are enzymes that are classified into EC2.7.1.40, and catalyze the reaction that dephosphorylates phosphoenolpyruvate to convert it to pyruvic acid and ATP. Specific examples include PykF (NCBI-Protein ID: NP_416191) and PykA (NCBI-Protein ID: NP_416368) derived from *Escherichia coli* str. K-12 substr. MG1655, and PykF (SEQ ID NO: 7) and PykA (SEQ ID NO: 8) derived from *Serratia grimesii* NBRC13537.

In the case where the microorganism used in the present invention has two or more genes encoding pyruvate kinases, it is preferable to reduce the functions of all of the pyruvate kinases. Whether or not the polypeptide encoded by a gene possessed by the microorganism used in the present invention is a pyruvate kinase can be determined by performing BLAST search on the public database in NCBI, KEGG, or the like.

The phosphotransferase system (PTS) is a main mechanism to uptake carbohydrates, such as hexose, hexitol, and disaccharides, into the inside of cells. Through the PTS, carbohydrates, once uptaken into cells, are converted into phosphate esters, while phosphoenolpyruvate (PEP) as a phosphate donor is converted into pyruvic acid.

The PTS enzymes are composed of two common enzymes, phosphoenolpyruvate sugar phosphotransferase system I and phospho carrier protein HPr, that function irrespective of the types of carbohydrates, and membrane-bound sugar specific permeases (enzyme II) specific for particular carbohydrates. The enzyme II is composed of sugar-specific IIA, IIB, and IIC components. The enzymes in the enzyme II exist independently or in conjugation as a multi-domain protein depending on biological species. In microorganisms, phosphoenolpyruvate sugar phosphotransferase system I is encoded by the ptsI gene, phospho carrier protein HPr is encoded by the ptsH gene, glucose-specific IIA is encoded by the err gene, and glucose-specific IIB and IIC are encoded by the ptsG gene.

The enzymes encoded by the ptsG gene are classified into EC2.7.1.199, called protein-Npi-phosphohistidine-D-glucose phosphotransferase, including PtsG derived from *Escherichia coli* str. K-12 substr. MG1655 (NCBI-Protein ID: NP_415619) and PtsG derived from *Serratia grimesii* NBRC13537 (SEQ ID NO: 9). Whether or not the polypeptides encoded by genes possessed by the microorganism used in the present invention are PTS enzymes can be determined by performing BLAST search on the website of NCBI, KEGG, or the like.

In the present invention, the function of one of the PTS enzymes may be reduced, or two or more functions may be reduced. Any of the functions of the PTS enzymes may be reduced, but preferably the functions involved in the uptake of glucose are reduced, and particularly preferably the function of PtsG is reduced. Specific example of the ptsG gene include ptsG derived from *Escherichia coli* str. K-12 substr. MG1655 (NCBI Gene ID: 945651) and ptsG derived from *Serratia grimesii* strain NBRC13537 (SEQ ID NO: 10).

As described later, *Escherichia coli* is a microorganism having an ability to produce 3-hydroxyadipic acid and $\alpha$-hydromuconic acid. JP 2008-527991 A describes that mutant strain of *Escherichia coli* with deletion of pykF and pykA encoding pyruvate kinase and the ptsG gene encoding phosphotransferase system is prepared, and that culturing of the mutant strain under anaerobic conditions results in increased yield of succinic acid and decreased yields of acetic acid and ethanol. Here, acetic acid and ethanol are compounds that are metabolized from acetyl-CoA as shown in the metabolic pathway in Scheme 2. Thus, in JP 2008-527991 A, it is estimated that the deletion of the ptsG, pkF, and pykA genes in *Escherichia coli* reduces the supply of acetyl-CoA resulted in reduction of the yields of acetic acid and ethanol.

On the other hand, 3-hydroxyadipic acid and/or $\alpha$-hydromuconic acid produced by the method of the present invention are compounds, as described above, that are metabolized from 3-oxoadipyl-CoA generated from acetyl-CoA and succinyl-CoA via a plurality of reactions. Therefore, considering the description in JP 2008-527991 A, it is expected that deletion of the genes of pyruvate kinase and phosphotransferase system reduces the supply of acetyl-CoA and

reduce the yield(s) of 3-hydroxyadipic acid and/or α-hydromuconic acid. However, contrary to the expectation described above, the genetically modified microorganism in the present invention, in which the reaction to generate acetyl-CoA from pyruvic acid is enhanced and the reaction that reduces 3-oxoadipyl-CoA to generate 3-hydroxyadipyl-CoA is enhanced, shows improved yield(s) of 3-hydroxyadipic acid and/or α-hydromuconic acid when the function of pyruvate kinase and/or phosphotransferase system is reduced.

In addition, it is preferred in the present invention that the phosphoenolpyruvate (PEP) carboxykinase (Pck) reaction be enhanced in order to enhance the ability to produce 3-hydroxyadipic acid and/or α-hydromuconic acid. Enhancing the PEP carboxykinase reaction includes, for example, enhancing the activity of the enzyme that catalyzes the reaction. Examples of the method to enhance the activity of the enzyme include introducing the enzyme gene into a host microorganism from outside the microorganism; increasing the copy number of the gene; and modifying the promoter region or the ribosome binding sequence upstream of the coding region of the gene. These methods may be performed solely or in combination, but it is preferred that the enzyme gene is introduced into host microorganisms from outside the microorganisms by the method described in WO 2019/107516 (US 2020/291435 A1). WO 2019/107516 (US 2020/291435 A1) is incorporated herein by reference.

PEP carboxykinase is an enzyme that is classified into EC4.1.1.49 and catalyzes the reaction to generate oxaloacetic acid and ATP from PEP, carbon dioxide, and ADP. Specific examples include Pck derived from *Escherichia coli* str. K-12 substr. MG1655 (NCBI-Protein ID: NP_417862), and PckA_1 (SEQ ID NO: 11) and PckA_2 (SEQ ID NO: 12) derived from *Serratia grimesii* strain NBRC13537.

PEP carboxykinase is physiologically responsible for the major reaction in the production of glucose from fatty acid in gluconeogenesis. The reaction catalyzed by phosphoenolpyruvate carboxykinase is reversible, but in the production of 3-hydroxyadipic acid and/or α-hydromuconic acid, the reaction proceeds in the direction in which phosphoenolpyruvate and carbon dioxide are converted to oxaloacetic acid.

Whether or not the polypeptide encoded by an enzyme gene used in the present invention is phosphoenolpyruvate carboxykinase can be determined by performing BLAST search on the website of NCBI, KEGG, or the like.

JP 2015-146810 A describes that deletion of the PEP carboxykinase gene is effective to prepare in silico microorganism strains that produce adipic acid from acetyl-CoA and succinyl-CoA in a high yield using a metabolism network model. In addition, JP 2015-504688 A describes that the activity of PEP carboxykinase is enhanced in order to increase the PEP pool in the production of muconic acid biosynthesized via PEP. Further, US 2017/0298363 A1 describes that the activity of PEP carboxykinase is enhanced in order to improve the availability of PEP in the production of adipic acid biosynthesized via PEP. Thus, based on the description that the reaction catalyzed by PEP carboxykinase proceeds in the direction in which PEP is produced from oxaloacetic acid, it is thought that those skilled in the art will expect that enhancing the enzyme activity by increasing the expression of the PEP carboxykinase gene results in lower yields of 3-hydroxyadipic acid and/or α-hydromuconic acid. However, contrary to the expectation described above, it was found that culturing of the genetically modified microorganism having an ability to produce 3-hydroxyadipic acid and/or α-hydromuconic acid in the present invention, in which the reaction to generate acetyl-CoA from pyruvic acid is enhanced, the reaction that reduces 3-oxoadipyl-CoA to generate 3-hydroxyadipyl-CoA is enhanced, as well as the function of pyruvate kinase and/or phosphotransferase system is reduced, and the phosphoenolpyruvate carboxykinase reaction is enhanced, results in improved production of 3-hydroxyadipic acid and/or α-hydromuconic acid.

It is also preferred in the present invention that the reaction to reduce 3-oxoadipyl-CoA to generate 3-hydroxyadipyl-CoA be enhanced. Enhancing the reaction that reduces 3-oxoadipyl-CoA to generate 3-hydroxyadipyl-CoA includes, for example, enhancing the activity of the enzyme that catalyzes the reaction. Examples of the method to enhance the activity of the enzyme include introducing the enzyme gene into host microorganisms from outside the microorganisms; increasing the copy number of the gene; and modifying the promoter region or the ribosome binding sequence upstream of the coding region of the gene. These methods may be performed solely or in combination, but it is preferred that the enzyme gene is introduced into a host microorganism from outside the microorganism by the method described in WO 2019/107516 (US 2020/291435 A1).

Specific examples of the 3-oxoadipyl-CoA reductase that catalyzes the reaction where 3-oxoadipyl-CoA is reduced to generate 3-hydroxyadipyl-CoA include enzymes classified into EC1.1.1.3 5 as 3-hydroxyacyl-CoA dehydrogenases; enzymes classified into EC1.1.1 .157 as 3-hydroxybutyryl-CoA dehydrogenases; and enzymes having 70% or more sequence identity with any amino acid sequence of SEQ ID NOs: 1 to 6 and 213 and having a 3-oxoadipyl-CoA reductase activity disclosed in WO 2019/107516 (US 2020/291435 A1). Specific examples of the enzyme that catalyzes the reaction where 3-oxoadipyl-CoA is reduced to generate 3-hydroxyadipyl-CoA include PaaH derived from *Pseudomonas putida* strain KT2440 (NCBI-Protein ID: NP_745425.1), PaaH derived from *Escherichia coli* str. K-12 substr. MG1655 (NCBI-Protein ID: NP_415913.1), DcaH derived from *Acinetobacter baylyi* ADP1 (NCBI-Protein ID: CAG68533.1), PaaH derived from *Serratiaplymuthica* NBRC102599 (NCBI-Protein ID: WP_063197120), and a polypeptide derived from *Serratia marcescens* strain ATCC13880 (NCBI-Protein ID: KFD11732.1).

It is preferred that the genetically modified microorganism of the present invention be a microorganism that does not undergo glucose metabolism via the phosphoketolase pathway from the viewpoint of productivity of 3-hydroxy-

adipic acid and/or $\alpha$-hydromuconic acid. Such a microorganism exists in the nature and can be preferably used as a host for creation of the genetically microorganism of the present invention that does not undergo glucose metabolism via the phosphoketolase pathway. A microorganism that does not undergo glucose metabolism via the phosphoketolase pathway may also be created by a method comprising creating the genetically modified microorganism of the present invention and then knocking out the phosphoketolase gene of the microorganism by a method well known by those skilled in the art.

Microorganisms originally having an ability to produce 3-hydroxyadipic acid include the following microorganisms:

- the genus *Escherichia,* such as *Escherichia fergusonii* and *Escherichia coli;*
- the genus *Serratia,* such as *Serratia grimesii*, *Serratia ficaria*, *Serratia fonticola*, *Serratia odorifera*, *Serratia plymuthica*, *Serratia entomophila*, and *Serratia nematodiphila*;
- the genus *Pseudomonas*, such as *Pseudomonas chlororaphis, Pseudomonas putida, Pseudomonas azotoformans*, and *Pseudomonas chlororaphis* subsp. aureofaciens;
- the genus *Hafnia,* such as *Hafnia alvei*;
- the genus *Corynebacterium*, such as *Corynebacterium acetoacidophilum*, *Corynebacterium acetoglutamicum*, *Corynebacterium ammoniagenes*, and *Corynebacterium glutamicum;*
- the genus *Bacillus*, such as *Bacillus badius*, *Bacillus megaterium*, and *Bacillus roseus;*
- the genus *Streptomyces*, such as *Streptomyces vinaceus*, *Streptomyces karnatakensis*, and *Streptomyces olivaceus*;
- the genus *Cupriavidus*, such as *Cupriavidus metallidurans*, *Cupriavidus necator,* and *Cupriavidus oxalaticus*;
- the genus *Acinetobacter,* such as *Acinetobacter baylyi* and *Acinetobacter radioresistens;*
- the genus *Alcaligenes,* such as *Alcaligenes, faecalis;*
- the genus *Nocardioides*, such as *Noc$\alpha$rdioides albus;*
- the genus *Brevibacterium,* such as *Brevibacterium iodinum*;
- the genus *Delftia*, such as *Delftia acidovorans*;
- the genus *Shimwellia*, such as *Shimwellia blattae*;
- the genus *Aerobacter,* such as *Aerobacter clo$\alpha$cae;* and
- the genus *Rhizobium*, such as *Rhizobium radiobacter.*

Among the microorganisms originally having an ability to produce 3-hydroxyadipic acid, the microorganisms belonging to the genus *Escherichia, Serratia, Hafnia,* or *Corynebacterium,* such as *Corynebacterium acetoacidophilum*, *Corynebacterium acetoglutamicum*, *Corynebacterium ammoniagenes,* and *Corynebacterium glutamicum*, *Brevibacterium*, *Shiniwellia*, or *Aerobacter*, which are microorganisms that do not undergo glucose metabolism via the phosphoketolase pathway, are preferably used in the present invention, and microorganisms belonging to the genus *Escherichia* or *Serratia* are more preferably used.

Microorganisms that are speculated to originally have the ability to produce $\alpha$-hydromuconic acid include the following microorganisms:

- the genus *Escherichia*, such as *Escherichia fergusonii* and *Escherichia coli;*
- the genus *Serratia,* such as *Serratia grimesii, Serratia ficaria*, *Serratia fonticola*, *Serratia odorifera*, *Serratia plymuthica*, *Serratia entomophila*, and *Serratia nematodiphila*;
- the genus *Pseudomonas,* such as *Pseudomonas fluorescens, Pseudomonas pulida*, *Pseudomonas azotoformans*, and *Pseudomonas chlororaphis* subsp. *aureofaciens*;
- the genus *Hafnia*, such as *Hafnia alvei*;
- the genus *Bacillus,* such as *Bacillus badius;*
- the genus *Cupriavidus*, such as *Cupriavidus metallidurans*, *Cupriavidus numazuensis*, and *Cupriavidus oxalaticus*;
- the genus *Acinetobacter,* such as *Acinetobacter baylyi* and *Acinetobacter radioresistens*;
- the genus *Alcaligenes*, such as *Alcaligenes faecalis*;
- the genus *Delftia*, such as *Delftia acidovorans*; and
- the genus *Shimwellia,* such as *Shimwellia blattae.*

Among the microorganisms originally having an ability to produce $\alpha$-hydromuconic acid, the microorganisms belonging to the genus *Escherichia, Serratia, Hafnia,* or *Shimwellia*, which are microorganisms that do not undergo glucose metabolism via the phosphoketolase pathway, are preferably used in the present invention, and a microorganism belonging to the genus *Escherichia* or *Serratia* is more preferably used.

When the genetically modified microorganism of the present invention does not originally have the ability to produce 3-hydroxyadipic acid, an appropriate combination of nucleic acids encoding the enzymes that catalyze the

reactions A, B, and D may be introduced into the microorganisms to impart the ability to produce them. On the other hand, when the genetically modified microorganism of the present invention does not originally have the ability to produce α-hydromuconic acid, an appropriate combination of nucleic acids encoding the enzymes that catalyze the reactions A, B, C, and E may be introduced into the microorganism to impart the ability to produce them.

In the present invention, the microorganisms that can be used as hosts to obtain the genetically modified microorganisms are not limited to particular microorganisms, provided that they can be genetically modified, and may be microorganisms with or without the ability to produce 3-hydroxyadipic acid and/or α-hydromuconic acid, and are preferably microorganisms belonging to the genus *Escherichia*, *Serratia, Hafnia, Pseudomonas, Corynebacterium, Bacillus, Streptomyces, Cupriavidus, Acinetobacter, Alcaligenes, Brevibacterium, Delftia*, *Shimwellia*, *Aerobacter, Rhizobium*, *Thermobifida*, *Clostridium*, *Schizosaccharomyces*, *Kluyveromyces, Pichia,* or *Candida*, more preferably microorganisms belonging to the genus *Escherichia, Serratia*, *Hafnia,* or *Pseudomonas,* and particularly preferably microorganisms belonging to the genus *Escherichia,* or *Serratia.*

The method to introduce a gene to create the genetically modified microorganism of the present invention is not limited to a particular method, and examples of the method that can be used include a method in which the gene incorporated in an expression vector capable of autonomous replication in a microorganism is introduced into a host microorganism, and a method in which the gene is integrated into the genome of a microorganism.

One or more genes may be introduced. Moreover, introduction of a gene and enhancement of expression may be combined.

When a gene expressed in the present invention is integrated into an expression vector or the genome of a host microorganism, the nucleic acid which is integrated into the expression vector or the genome is preferably composed of a promoter, a ribosome-binding sequence, a gene to be expressed, and a transcription termination sequence. In addition, the nucleic acid may also contain a gene that controls the activity of the promoter.

The promoter used in the present invention is not limited to a particular promoter, provided that the promoter drives expression of the gene in the host microorganism; examples of the promoter include gap promoter, trp promoter, lac promoter, tac promoter, and T7 promoter.

In cases where an expression vector is used in the present invention to introduce genes or to enhance the expression of genes, the expression vector is not limited to a particular vector, provided that the vector is capable of autonomous replication in the microorganism; examples of the vector include pBBR1MCS vector, pBR322 vector, pMW vector, pET vector, pRSF vector, pCDF vector, pACYC vector, and derivatives of the above vectors.

In cases where a nucleic acid for genome integration is used in the present invention to introduce the gene or to enhance the expression of the gene, the nucleic acid for genome integration is introduced by site-specific homologous recombination. The method for site-specific homologous recombination is not limited to a particular method, and examples of the method include a method in which λ Red recombinase and FLP recombinase are used (Proc Natl Acad Sci U.S.A. 2000 Jun 6; 97 (12): 6640-6645.), and a method in which λ Red recombinase and the sacB gene are used (Biosci Biotechnol Biochem. 2007 Dec; 71 (12): 2905-11.).

The method of introducing the expression vector or the nucleic acid for genome integration is not limited to a particular method, provided that the method is for introduction of a nucleic acid into a microorganism; examples of the method include the calcium ion method (Journal of Molecular Biology, 53,159 (1970)), and electroporation (NM Calvin, PC Hanawalt. J. Bacteriol, 170 (1988), pp. 2796-2801).

The genetically modified microorganism of the present invention is cultured in a culture medium, preferably a liquid culture medium, containing a carbon source as a material for fermentation which can be used by ordinary microorganisms. The culture medium used contains, in addition to the carbon source that can be used by the genetically modified microorganism, appropriate amounts of a nitrogen source, inorganic salts, and, if necessary, organic trace nutrients such as amino acids and vitamins. Any of natural and synthetic culture medium can be used as long as the medium contains the above-described nutrients.

The material for fermentation is a material that can be metabolized by the genetically modified microorganism. The term "metabolize" refers to conversion of a chemical compound, which a microorganism has taken up from the extracellular environment or intracellularly generated from a different chemical compound, to another chemical compound through an enzymatic reaction. Sugars can be suitably used as the carbon source. Specific examples of the sugars include monosaccharides, such as glucose, sucrose, fructose, galactose, mannose, xylose, and arabinose; disaccharides and polysaccharides formed by linking these monosaccharides; and saccharified starch solution, molasses, and saccharified solution from cellulose-containing biomass, each containing any of those saccharides.

The above-listed carbon sources may be used individually or in combination, and culturing in a culture medium containing glucose is particularly preferred. When a carbon source is added, the concentration of the carbon source in the culture medium is not particularly limited, and can be appropriately selected depending on the type of the carbon source, or the like. A preferred concentration of glucose is from 5 to 300 g/L.

As the nitrogen source used for culturing the genetically modified microorganism, for example, ammonia gas, aqueous ammonia, ammonium salts, urea, nitric acid salts, other supportively used organic nitrogen sources, such as

oil cakes, soybean hydrolysate, casein degradation products, other amino acids, vitamins, corn steep liquor, yeast or yeast extract, meat extract, peptides such as peptone, and bacterial cells and hydrolysate of various fermentative bacteria can be used. The concentration of the nitrogen source in the culture medium is not particularly limited, and is preferably from 0.1 to 50 g/L.

As the inorganic salts used for culturing the genetically modified microorganism, for example, phosphoric acid salts, magnesium salts, calcium salts, iron salts, and manganese salts can be appropriately added to the culture medium and used.

The culture conditions for the genetically modified microorganism to produce 3-hydroxyadipic acid and/or α-hydromuconic acid are set by appropriately adjusting or selecting, for example, the culture medium with the above composition, culture temperature, stirring speed, pH, aeration rate, and inoculation amount, depending on the species of the genetically modified microorganism and external conditions, and the like.

The pH range in the culturing is not particularly limited, provided that the genetically modified microorganism can grow, and is preferably from pH5 to 8, more preferably from pH5.5 to 6.8.

The range of the aeration rate condition in the culturing is not particularly limited, provided that 3-hydroxyadipic acid and/or α-hydromuconic acid can be produced, and it is preferred that oxygen remain in the gas and/or liquid phase in the culture vessel at least at the start of the culturing in order to allow for good growth of the microbial mutant.

In cases where foam is formed in a liquid culture, an antifoaming agent such as a mineral oil, silicone oil, or surfactant may be appropriately added to the culture medium.

After a recoverable amount of 3-hydroxyadipic acid and/or α-hydromuconic acid is produced during culturing of the microorganism, the produced products can be recovered. The produced products can be recovered, for example isolated, according to a commonly used method, in which the culturing is stopped once a product of interest is accumulated to an appropriate level, and the fermentation product is collected from the culture. Specifically, the products can be isolated from the culture by separation of bacterial cells through, for example, centrifugation or filtration prior to, for example, column chromatography, ion exchange chromatography, activated charcoal treatment, crystallization, membrane separation, or distillation. More specifically, examples include, but are not limited to, a method in which an acidic component is added to salts of the products, and the resulting precipitate is collected; a method in which water is removed from the culture by concentration using, for example, a reverse osmosis membrane or an evaporator to increase the concentrations of the products and the products and/or salts of the products are then crystallized and precipitated by cooling or adiabatic crystallization to recover the crystals of the products and/or salts of the products by, for example, centrifugation or filtration; and a method in which an alcohol is added to the culture to produce esters of the products and the resulting esters of the products are subsequently collected by distillation and then hydrolyzed to recover the products. These recovery methods can be appropriately selected and optimized depending on, for example, physical properties of the products.

## EXAMPLES

The present invention will now be specifically described by way of Examples.

### Reference Example 1

Production of a plasmid expressing an enzyme catalyzing a reaction to generate 3OA-CoA and coenzyme A from acetyl-CoA and succinyl-CoA (the reaction A) and an enzyme catalyzing a reaction to generate 3HA-CoA from 3OA-CoA (the reaction B), and a reaction to generate 3-hydroxyadipic acid from 3HA-CoA (the reaction D) and a reaction to generate α-hydromuconic acid from HMA-CoA (the reaction E)

The pBBR1MCS-2 vector (ME Kovach, (1995), Gene 166: 175-176), capable of autonomous replication in *E. coli,* was cleaved with XhoI to obtain pBBR1MCS-2/XhoI. To integrate a constitutive expression promoter into the vector, primers (SEQ ID NOs: 14 and 15) were designed for use in amplification of an upstream 200-b region (SEQ ID NO: 13) of gapA (NCBI Gene ID: NC_000913.3) by PCR using the genomic DNA of *Escherichia coli* str. K-12 substr. MG1655 as a template, and a PCR reaction was performed in accordance with routine procedures. The resulting fragment and the pBBR1MCS-2/XhoI were ligated together using the In-Fusion HD Cloning Kit (manufactured by Takara Bio Inc.), and the resulting plasmid was introduced into *E. coli* strain DH5α. The nucleotide sequence on the plasmid extracted from the obtained recombinant *E. coli* strain was confirmed in accordance with routine procedures, and the plasmid was designated as pBBR1MCS-2::Pgap. Then, the pBBR1MCS-2::Pgap was cleaved with Seal to obtain pBBR1MCS-2::Pgap/ScaI. To amplify a gene encoding an enzyme catalyzing the reaction A, primers (SEQ ID NOs: 16 and 17) were designed for use in amplification of the full length of the acyltransferase gene pcaF (NCBI Gene ID: 1041755) by PCR using the genomic DNA of *Pseudomonas putida* strain KT2440 as a template, and a PCR reaction was performed in accordance with routine procedures. The resulting fragment and the pBBR1MCS-2::Pgap/ScaI were ligated together

using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::AT. Then, the pBBR1MCS-2::AT was cleaved with HpaI to obtain pBBR1MCS-2::AT/HpaI. To amplify a gene encoding an enzyme catalyzing the reactions D and E, primers (SEQ ID NOs: 18 and 19) were designed for use in amplification of a continuous sequence including the full lengths of genes together encoding a CoA transferase, peal and pcaJ (NCBI Gene IDs: 1046613 and 1046612), by PCR using the genomic DNA of *Pseudomonas putida* strain KT2440 as a template, and a PCR reaction was performed in accordance with routine procedures. The resulting fragment and the pBBR1MCS-2::AT/HpaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCT.

The pBBR1MCS-2::ATCT was cleaved with Seal to obtain pBBR1MCS-2::ATCT/ScaI. Primers (SEQ ID NOs: 21 and 22) were designed for use in amplification of a nucleic acid represented by SEQ ID NO: 20 using the genomic DNA of *Serratia marcescens* strain ATCC13880 as a template, and a PCR reaction was performed in accordance with routine procedures. The resulting fragment and the pBBR1MCS-2::ATCT/ScaI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCTOR.

Example 1

Preparation of mutant *Serratia* microorganism with reduced function of pyruvate kinase

pykF and pykA genes coding for pyruvate kinases of *Serratia* microorganism were deleted to prepare a mutant *Serratia* microorganism with reduced function of the pyruvate kinase.

The methods of deleting pykF and pykA were performed according to the methods described in Proc Natl Acad Sci USA. 2000 Jun 6; 97(12): 6640-6645.

pykF deficiency

PCR was performed using pKD4 as a template and oligo DNAs represented by SEQ ID NOs: 23 and 24 as primers to obtain a PCR fragment for pykF deficiency. pKD46 which is a FRT recombinase expression plasmid, was introduced into a *Serratia grimesii* strain NBRC13537 to obtain an ampicillin-resistant strain. The resulting strain was seeded in 5 mL of LB medium containing 500 μg/mL ampicillin, and incubated with shaking at 30°C for 1 day. Thereafter, 0.5 mL of the culture was seeded in 50 mL of LB medium containing 500 μg/mL ampicillin and 50 mM arabinose, and incubated at 30°C for 2 hours with rotation. The culture was cooled on ice for 20 minutes, and then the bacterial cells were washed with 10%(w/w) glycerol 3 times. The washed pellet was resuspended in 100 μL of 10%(w/w) glycerol, mixed with 5 μL of the PCR fragment, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and cultured at 30°C for 2 hours with shaking. The whole culture was applied to LB agar medium containing 25 μg/mL kanamycin and incubated at 30°C for 1 day. The resulting kanamycin-resistant strain was subjected to colony direct PCR to confirm that the gene of interest was deleted and the kanamycin resistance gene was inserted based on the band lengths. The primers used were oligo DNAs represented by SEQ ID NOs: 25 and 27.

Thereafter, the kanamycin-resistant strain was seeded in 5 mL of LB medium and subcultured twice at 37°C to allow for loss of pKD46, thereby obtaining ampicillin-sensitive strain. pCP20 was introduced into the ampicillin-sensitive strain to again obtain ampicillin-resistant strain. The resulting strain was cultured at 40°C and then subjected to colony direct PCR to confirm that the kanamycin resistance gene was lost based on the band lengths. The primers used were oligo DNAs represented by SEQ ID NOs: 26 and 27. The kanamycin-sensitive strain was seeded in 5 mL of LB medium and subcultured twice at 37°C to allow for loss of pCP20. The resulting strain was designated as SgΔPf.

pykA deficiency

PCR was performed using pKD4 as a template and oligo DNAs represented by SEQ ID NOs: 28 and 29 as primers to obtain a PCR fragment for pykA deficiency.

The same method as the preparation of the pykF-deficient strain was performed to delete pykA of *Serratia grimesii* strain NBRC13537ΔpykF. pKD46 was introduced into the strain, and then the PCR fragment for pykA deficiency was introduced. The resulting kanamycin-resistant strain was subjected to colony direct PCR to confirm that the gene of interest was deleted and the kanamycin resistance gene was inserted based on the band lengths. The primers used were oligo DNAs represented by SEQ ID NOs: 25 and 31.

Thereafter, pKD46 was allowed to be lost to obtain an ampicillin-sensitive strain. pCP20 was introduced into the ampicillin-sensitive strain to again obtain an ampicillin-resistant strain. The resulting strain was subjected to colony direct PCR to confirm that the kanamycin resistance gene was lost based on the band lengths. The primers used were oligo DNAs represented by SEQ ID NOs: 30 and 31. The kanamycin-sensitive strain was allowed to lose pCP20. The resulting strain was designated as SgΔPP.

Example 2

Preparation of mutant *Serratia* microorganism with reduced function of phosphotransferase system

ptsG gene coding for phosphotransferase of *Serratia* microorganism was deleted to prepare a mutant *Serratia* microorganism with reduced function of pyruvate phosphotransferase system.

PCR was performed using pKD4 as a template and oligo DNAs represented by SEQ ID NOs: 32 and 33 as primers to obtain a PCR fragment for ptsG deficiency. pKD46 was introduced into *Serratia grimesii* NBRC13537 and SgΔPP, and then the PCR fragment for ptsG deficiency was introduced. The resulting kanamycin-resistant strains were subjected to colony direct PCR to confirm that the gene of interest was deleted and the kanamycin resistance gene was inserted based on the band lengths. The primers used were oligo DNAs represented by SEQ ID NOs: 25 and 35.

Thereafter, pKD46 was allowed to be lost to obtain ampicillin-sensitive strains. pCP20 was introduced into the ampicillin-sensitive strains to again obtain ampicillin-resistant strains. The resulting strains were subjected to colony direct PCR to confirm that the kanamycin resistance gene was lost based on the band lengths. The primers used were oligo DNAs represented by SEQ ID NOs: 34 and 35. The kanamycin-sensitive strains were allowed to lose pCP20. The resulting strains were designated as SgΔG and SgΔPPG, respectively.

Example 3

Preparation of mutant *Serratia* microorganism with reduced function of the pyruvate dehydrogenase complex transcriptional repressor

pdhR, a gene coding for the pyruvate dehydrogenase complex transcriptional repressor of *Serratia* microorganism (SEQ ID NO: 36) was deleted to prepare mutant *Serratia* microorganism with increased expression of PDHc.

PCR was performed using pKD4 as a template and oligo DNAs represented by SEQ ID NOs: 37 and 38 as primers to obtain a PCR fragment for pdhR deficiency. pKD46 was individually introduced into SgΔPP, SgΔG, and SgΔGPP, and then the PCR fragment for pdhR deficiency was introduced. The resulting kanamycin-resistant strains were subjected to colony direct PCR to confirm that the gene of interest was deleted and the kanamycin resistance gene was inserted based on the band lengths. The primers used were oligo DNAs represented by SEQ ID NOs: 25 and 40.

Thereafter, pKD46 was allowed to be lost to obtain ampicillin-sensitive strains. pCP20 was introduced into the ampicillin-sensitive strains to again obtain ampicillin-resistant strains. The resulting strains were subjected to colony direct PCR to confirm that the kanamycin resistance gene was lost based on the band lengths. The primers used were oligo DNAs represented by SEQ ID NOs: 39 and 40. The kanamycin-sensitive strains were allowed to lose pCP20. The resulting strains were designated as SgΔPPR, SgΔGR, and SgΔGPPR, respectively.

Example 4

Preparation of mutant *Serratia* microorganism with reduced function of the pyruvate dehydrogenase complex transcriptional repressor and with introduced plasmid expressing enzymes that catalyze reactions A, B, D and E

The plasmids prepared in Reference Example 1 were individually introduced into the strains prepared in Example 3 to prepare mutant *Serratia* microorganisms.

SgΔPPR, SgΔGR, and SgΔGPPR were respectively seeded in 5 mL of LB medium and incubated with shaking at 30°C for 1 day. Subsequently, 0.5 mL each of the cultures was seeded in 5 mL of LB medium, and incubated at 30°C for 2 hours with shaking. The cultures were cooled on ice for 20 minutes, and then the bacterial cells were washed with 10%(w/w) glycerol 3 times. The washed pellets were resuspended in 100 μL of 10%(w/w) glycerol, mixed with 1 μL of pBBR1MCS-2::ATCTOR, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 1 hour with shaking. Subsequently, 50 μL of the cultures were applied to LB agar medium containing 25 μg/mL kanamycin and incubated at 30°C for 1 day. The resulting strains were designated as SgΔPPR/3HA, SgΔGR/3HA, and SgΔGPPR/3HA, respectively.

Example 5

Test for production of 3-hydroxyadipic acid and α-hydromuconic acid using mutant *Serratia* microorganism with reduced function of the pyruvate dehydrogenase complex transcriptional repressor and with introduced plasmid expressing enzymes that catalyze reactions A, B, D and E

The mutant *Serratia* microorganisms prepared in Example 4 were used to perform a test for production of 3-hydroxyadipic acid and α-hydromuconic acid.

A loopful of each mutant prepared in Example 2 was inoculated into 5 mL (φ18-mm glass test tube, aluminum plug) of the culture medium I (10 g/L Bacto Tryptone (manufactured by Difco Laboratories), 5 g/L Bacto Yeast Extract (manufactured by Difco Laboratories), 5 g/L sodium chloride, 25 μg/mL kanamycin) adjusted to pH 7, and incubated at 30°C for 24 hours with shaking at 120 $min^{-1}$. Subsequently, 0.25 mL of the culture fluid was added to 5 mL (φ18-mm glass test tube, aluminum plug) of the culture medium II (50 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract, 25 μg/mL kanamycin) adjusted to pH 6.5, and incubated at 30°C with shaking.

Quantitative analyses of substrate and product

The supernatant separated from bacterial cells by centrifugation of the culture fluid was processed by membrane treatment using Millex-GV (0.22 μm; PVDF; manufactured by Merck KGaA), and the resulting filtrate was analyzed according to the following method to measure the concentrations of 3-hydroxyadipic acid, α-hydromuconic acid, and other products accumulated in the culture supernatant, and of sugars remaining in the culture medium without being used. Additionally, the yields of 3-hydroxyadipic acid and α-hydromuconic acid calculated based on the results according to the following Formula (1) are presented in Table 1.

$$\text{Yield (\%)} = \text{amount of formed product (mol)} / \text{amount of consumed sugar (mol)} \times 100 \quad \text{Formula (1)}$$

(Quantitative analyses of 3-hydroxyadipic acid and α-hydromuconic acid by LC-MS/MS)

·HPLC: 1290 Infinity (manufactured by Agilent Technologies, Inc.)
Column: Synergi hydro-RP (manufactured by Phenomenex Inc.), length: 100 mm, internal diameter: 3 mm, particle size: 2.5 μm
Mobile phase: 0.1% aqueous formic acid solution / methanol = 70/30
Flow rate: 0.3 mL/min
Column temperature: 40°C
I,C detector: 1260DAD VL+ (210 nm)
·MS/MS: Triple-Quad LC/MS (manufactured by Agilent Technologies, Inc.)
Ionization method: ESI in negative mode.

Quantitative analyses of organic acids by HPLC

•HPLC:LC-10A (manufactured by Shimadzu Corporation)

Column: Shim-pack SPR-H (manufactured by Shimadzu GLC Ltd.), length: 250 mm, inner diameter: 7.8 mm, particle size: 8 μm
Shim-pack SCR-101H (manufactured by Shimadzu GLC Ltd.), length: 250 mm, inner diameter: 7.8 mm, particle size: 10 μm
Mobile phase: 5 mM p-toluenesulfonic acid
Reaction solution: 5 mM p-toluenesulfonic acid, 0.1 mM EDTA, 20 mM Bis-Tris Flow rate: 0.8 mL/min

Column temperature: 45°C
Detector: CDD-lOAvp (manufactured by Shimadzu Corporation)

Quantitative analyses of sugars by HPLC

•HPLC: Shimadzu Prominence (manufactured by Shimadzu Corporation)

Column: Shodex Sugar SH101 1 (manufactured by Showa Denko K.K.), length: 300 mm, internal diameter: 8 mm, particle size: 6 μm
Mobile phase: 0.05 M aqueous sulfuric acid solution
Flow rate: 0.6 mL/min
Column temperature: 65°C
Detector: RID-10A (manufactured by Shimadzu Corporation)

Reference Example 2

Preparation of mutant *Serratia* microorganism without reduction of function of the pyruvate dehydrogenase complex transcriptional repressor and with introduced plasmid expressing enzymes that catalyze reactions A, B, D and E

The plasmids prepared in Reference Example 1 were individually introduced into the strains prepared in Examples 1 and 2 in the same manner as in Example 4 to prepare mutant *Serratia* microorganisms. The resulting strains were designated as SgΔPP/3HA, SgΔG/3HA, and SgΔGPP/3HA, respectively.

Comparative Example 1

Test for production of 3-hydroxyadipic acid and α-hydromuconic acid using mutant *Serratia* microorganism without reduction of function of the pyruvate dehydrogenase complex transcriptional repressor and with introduced plasmid expressing enzymes that catalyze reactions A, B, D and E

The mutant *Serratia* microorganisms prepared in Reference Example 2 were cultured in the same manner as in Example 5. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 1.

As seen from Table 1, the mutant *Serratia* microorganisms with reduced function of the pyruvate dehydrogenase complex transcriptional repressor and with introduced plasmid expressing enzymes that catalyze reactions A, B, D and E showed improved yields of 3-hydroxyadipic acid and α-hydromuconic acid.

[Table 1]

| | strain | 3 HA yield (%) | HMA yield (%) |
|---|---|---|---|
| Comparative Example 1 | SgΔPP/3HA | 4.73 | 0.104 |
| | SgΔG/3HA | 0.934 | 0.058 |
| | SgΔGPP/3HA | 2.43 | 0.126 |
| Example 5 | SgΔPPR/3HA | 5.81 | 0.160 |
| | SgΔGR/3HA | 1.69 | 0.066 |
| | SgΔGPPR/3HA | 14.4 | 0.311 |

Example 6

Preparation of mutant *Escherichia* microorganism with reduced function of pyruvate kinase

pykF (NCBI Gene ID: 946179) and pykA (NCBI Gene ID:946527) genes coding for pyruvate kinases of *Es-*

*cherichia* microorganism were deleted to prepare mutant an *Escherichia* microorganism with reduced function of the pyruvate kinases.

The methods of deleting pykF and pykA were performed according to the methods described in Proc Natl Acad Sci USA. 2000 Jun 6; 97(12): 6640-6645.

pykF deficiency

PCR was performed using pKD4 as a template and oligo DNAs represented by SEQ ID NOs: 41 and 42 as primers to obtain PCR fragments for pykF deficiency. pKD46 which is a FRT recombinase expression plasmid, was introduced into *Escherichia coli* str. K-12 substr. MG1655 to obtain an ampicillin-resistant strain. The resulting strain was seeded in 5 mL of LB medium containing 100 μg/mL ampicillin, and incubated at 30°C for 1 day with shaking. Thereafter, 0.5 mL of the culture was seeded in 50 mL of LB medium containing 100 μg/mL ampicillin and 50 mM arabinose, and incubated at 30°C for 2 hours with rotation. The culture was cooled on ice for 20 minutes, and then the bacterial cells were washed with 10%(w/w) glycerol 3 times. The washed pellet was resuspended in 100 μL of 10%(w/w) glycerol, mixed with 5 μL of the PCR fragment, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 2 hours with shaking. The whole culture was applied to LB agar medium containing 25 μg/mL kanamycin and incubated at 30°C for 1 day. The resulting kanamycin-resistant strain was subjected to colony direct PCR to confirm that the gene of interest was deleted and the kanamycin resistance gene was inserted based on the band lengths. The primers used were oligo DNAs represented by SEQ ID NOs: 25 and 44.

Thereafter, the kanamycin-resistant strain was seeded in 5 mL of LB medium and subcultured twice at 37°C to allow for loss of pKD46, thereby obtaining an ampicillin-sensitive strain. pCP20 was introduced into the ampicillin-sensitive strain to again obtain an ampicillin-resistant strain. The resulting strain was cultured at 40°C and then subjected to colony direct PCR to confirm that the kanamycin resistance gene was lost based on the band lengths. The primers used were oligo DNAs represented by SEQ ID NOs: 43 and 44. The kanamycin-sensitive strain was seeded in 5 mL of LB medium and subcultured twice at 37°C to allow for loss of pCP20. The resulting strain was designated as EcΔPf.

pykA deficiency

PCR was performed using pKD4 as a template and oligo DNAs represented by SEQ ID NOs: 45 and 46 as primers to obtain PCR fragments for pykA deficiency.

The same method as the preparation of the pykF-deficient strain was performed to delete pykA of *Escherichia coli* str. K-12 substr. MG1655ΔpykF. pKD46 was introduced into the strain, and then the PCR fragment for pykA deficiency was introduced. The resulting kanamycin-resistant strain was subjected to colony direct PCR to confirm that the gene of interest was deleted and the kanamycin resistance gene was inserted based on the band lengths. The primers used were oligo DNAs represented by SEQ ID NOs: 25 and 48.

Thereafter, pKD46 was allowed to be lost to obtain an ampicillin-sensitive strain. pCP20 was introduced into the ampicillin-sensitive strain to again obtain an ampicillin-resistant strain. The resulting strain was subjected to colony direct PCR to confirm that the kanamycin resistance gene was lost based on the band lengths. The primers used were oligo DNAs represented by SEQ ID NOs: 47 and 48. The kanamycin-sensitive strain was allowed to lose pCP20. The resulting strain was designated as EcΔPP.

Example 7

Preparation of mutant *Escherichia* microorganism with reduced function of phosphotransferase system

ptsG gene coding for phosphotransferase of an *Escherichia* microorganism was deleted to prepare mutant *Escherichia* microorganism with reduced function of pyruvate phosphotransferase system.

PCR was performed using pKD4 as a template and oligo DNAs represented by SEQ ID NOs: 49 and 50 as primers to obtain a PCR fragment for ptsG deficiency. pKD46 was introduced into *Escherichia coli* str. K-12 substr. MG1655 and EcΔPP, and then the PCR fragment for ptsG deficiency was introduced. The resulting kanamycin-resistant strains were subjected to colony direct PCR to confirm that the gene of interest was deleted and the kanamycin resistance gene was inserted based on the band lengths. The primers used were oligo DNAs represented by SEQ ID NOs: 25 and 52.

Thereafter, pKD46 was allowed to be lost to obtain ampicillin-sensitive strains. pCP20 was introduced into the ampicillin-sensitive strains to again obtain ampicillin-resistant strains. The resulting strains were subjected to colony direct PCR to confirm that the kanamycin resistance gene was lost based on the band lengths. The primers used were oligo DNAs represented by SEQ ID NOs: 51 and 52. The kanamycin-sensitive strains were allowed to lose pCP20. The

resulting strains were designated as EcΔG and EcΔPPG, respectively.

Example 8

Preparation of mutant *Escherichia* microorganism with reduced function of pyruvate dehydrogenase complex transcriptional repressor

pdhR which is a gene coding for the pyruvate dehydrogenase complex transcriptional repressor of *Escherichia* microorganisms (NCBI Gene ID: 944827) was deleted to prepare mutant an *Escherichia* microorganism with increased expression of PDHc.

PCR was performed using pKD4 as a template and oligo DNAs represented by SEQ ID NOs: 53 and 54 as primers to obtain a PCR fragment for pdhR deficiency. pKD46 was individually introduced into EcΔPP, EcΔG, and EcΔGPP, and then the PCR fragment for pdhR deficiency was introduced. The resulting kanamycin-resistant strains were subjected to colony direct PCR to confirm that the gene of interest was deleted and the kanamycin resistance gene was inserted based on the band lengths. The primers used were oligo DNAs represented by SEQ ID NOs: 25 and 56.

Thereafter, pKD46 was allowed to be lost to obtain ampicillin-sensitive strains. pCP20 was introduced into the ampicillin-sensitive strains to again obtain ampicillin-resistant strains. The resulting strains were subjected to colony direct PCR to confirm that the kanamycin resistance gene was lost based on the band lengths. The primers used were oligo DNAs represented by SEQ ID NOs: 55 and 56. The kanamycin-sensitive strains were allowed to lose pCP20. The resulting strains were designated as EcΔPPR, EcΔGR, and EcΔGPPR, respectively.

Example 9

Preparation of mutant *Escherichia* microorganism with reduced function of pyruvate dehydrogenase complex transcriptional repressor and with introduced plasmid expressing enzymes that catalyze reactions A, B, D and E

The plasmids prepared in Reference Example 1 were individually introduced into the strains prepared in Example 8 to prepare mutant *Escherichia*. microorganisms.

EcΔPPR, EcΔGR, and EcΔGPPR were respectively seeded in 5 mL of LB medium and incubated with shaking at 30°C for 1 day. Subsequently, 0.5 mL each of the cultures was seeded in 5 mL of LB medium, and incubated at 30°C for 2 hours with shaking. The cultures were cooled on ice for 20 minutes, and then the bacterial cells were washed with 10%(w/w) glycerol 3 times. The washed pellet was resuspended in 100 μL of 10%(w/w) glycerol, mixed with 1 μL of pBBR1MCS-2::ATCTOR, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 1 hour with shaking. Subsequently, 50 μL each of the cultures was applied to LB agar medium containing 25 μg/mL kanamycin and incubated at 30°C for 1 day. The resulting strains were designated as EcΔPPR/3HA, EcΔGR/3HA, and EcΔGPPR/3HA, respectively.

Example 10

Test for production of 3-hydroxyadipic acid and α-hydromuconic acid using mutant *Escherichia* microorganism with reduced function of pyruvate dehydrogenase complex transcriptional repressor and with introduced plasmid expressing enzymes that catalyze reactions A, B, D and E

The mutant *Escherichia* microorganisms prepared in Example 9 were cultured in the same manner as in Example 5. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 2.

Reference Example 3

Preparation of mutant *Escherichia* microorganism without reduction of function of pyruvate dehydrogenase complex transcriptional repressor and with introduced plasmid expressing enzymes that catalyze reactions A, B, D and E

The plasmids prepared in Reference Example 1 were individually introduced into the strains prepared in Examples 6 and 7 in the same manner as in Example 9 to prepare mutant *Escherichia* microorganisms. The resulting strains were designated as EcΔPP/3HA, EcΔG/3HA, and EcΔGPP/3HA, respectively.

Comparative Example 2

Test for production of 3-hydroxyadipic acid and α-hydromuconic acid using mutant *Escherichia* microorganism without reduction of function of pyruvate dehydrogenase complex transcriptional repressor and with introduced plasmid expressing enzymes that catalyze reactions A, B, D and E

The mutant *Escherichia* microorganisms prepared in Reference Example 3 were cultured in the same manner as in Example 5. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 2.

As seen from Table 2, the mutant *Escherichia* microorganisms with reduced function of the pyruvate dehydrogenase complex transcriptional repressor and with introduced plasmid expressing enzymes that catalyze reactions A, B, D and E showed improved yields of 3-hydroxyadipic acid and α-hydromuconic acid.

[Table 2]

| | strain | 3 HA yield (%) | HMA yield (%) |
|---|---|---|---|
| Comparative Example 2 | EcΔPP/3HA | 3.01 | 0.004 |
| | EcΔG/3HA | 4.38 | 0.013 |
| | EcΔGPP/3HA | 8.27 | 0.055 |
| Example 10 | EcΔPPR/3HA | 3.75 | 0.005 |
| | EcΔGR/3 HA | 5.24 | 0.014 |
| | EcΔGPPR/3HA | 13.5 | 0.087 |

Reference Example 4

Preparation of mutant *Escherichia* microorganism with reduced function of only pyruvate dehydrogenase complex transcriptional repressor

The same method as in Example 8 was performed to delete the pdhR gene from *Escherichia coli* str. K-12 substr. MG1655. The resulting strain was designated as EcΔR.

Reference Example 5

Preparation of mutant *Escherichia* microorganism with reduced function of only pyruvate dehydrogenase complex transcriptional repressor and with introduced plasmid expressing enzymes that catalyze reactions A, B, D and E, and mutant *Escherichia* microorganism with introduced plasmid expressing enzymes that catalyze reactions A, B, D and E

The plasmids prepared in Reference Example 1 were individually introduced into the strains EcΔR and *Escherichia coli* str. K-12 substr. MG1655 in the same manner as in Example 9 to prepare mutant *Escherichia* microorganisms. The resulting strains were designated as EcWT/3HA and EcΔK/3HA, respectively.

Reference Example 6

Test for production of 3-hydroxyadipic acid and α-hydromuconic acid using mutant *Escherichia* microorganism with reduced function of only pyruvate dehydrogenase complex transcriptional repressor and with introduced plasmid expressing enzymes that catalyze reactions A, B, D and E, and mutant *Escherichia* microorganism with introduced plasmid expressing enzymes that catalyze reactions A, B, D and E

The mutant *Escherichia* microorganisms prepared in Reference Example 5 were cultured in the same manner as in Example 5. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 3.

As seen from Table 3, the mutant *Escherichia* microorganisms with reduced function of only the pyruvate dehydrogenase complex transcriptional repressor and with introduced plasmid expressing enzymes that catalyze reactions A, B, D and E showed less yields of 3-hydroxyadipic acid and α-hydromuconic acid than the control strain.

[Table 3]

| | strain | 3 HA yield (%) | HMA yield (%) |
|---|---|---|---|
| Reference Example 6 | EcWT/3HA | 2.86 | 0.014 |
| | EcΔR/3HA | 2.47 | 0.008 |

Reference Example 7

Preparation of plasmid for expression of PDHc

The pMW119 expression vector (manufactured by Nippon Gene Co., Ltd.) capable of autonomous replication in *E. coli,* was cleaved with SacI to obtain pMW119/SacI. To integrate a constitutive expression promoter into the vector, primers (SEQ ID NOs: 57 and 58) were designed for use in amplification of an upstream 200-b region (SEQ ID NO: 13) of gapA (NCBI Gene ID: NC_000913.3) by PCR using the genomic DNA of *Escherichia coli* str. K-12 substr. MG1655 as a template, and a PCR reaction was performed in accordance with routine procedures. The resulting fragment and pMW119/SacI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant E. *coli* strain was confirmed in accordance with routine procedures, and the plasmid was designated as pMW119::Pgap. Then, the pMW119::Pgap was cleaved with SphI to obtain pMW119::Pgap/SphI. To amplify a gene encoding the pyruvate dehydrogenase complex, primers (SEQ ID NOs: 59 and 60) were designed for use in amplification of the region comprising the full lengths of aceE (NCBI Gene ID: 944834), aceF (NCBI Gene ID: 944794), and lpd (NCBI Gene ID: 944854) by PCR using the genomic DNA of *Escherichia coli* str. K-12 substr. MG1655 as a template, and a PCR reaction was performed in accordance with routine procedures. The resulting fragment and pMW119::Pgap/SphI were ligated together using the In-Fusion HD Cloning Kit (manufactured by Takara Bio Inc.), and the resulting plasmid was introduced into *E. coli* strain DH5α. The nucleotide sequence on the plasmid extracted from the obtained ampicillin-resistant strain was confirmed in accordance with routine procedures. The obtained plasmid was designated as "pMW119::PDHc."

Reference Example 8

Preparation of plasmid for expression of PFL

To amplify a gene encoding PFL, primers (SEQ ID NOs: 61 and 62) were designed for use in amplification of the region comprising the full lengths of pflB (NCBI Gene ID: 945514) and pflA (NCBI Gene ID: 945517) by PCR using the genomic DNA of *Escherichia coli* str. K-12 substr. MG1655 as a template, and a PCR reaction was performed in accordance with routine procedures. The resulting fragment and pMW119::Pgap/SphI were ligated together using the In-Fusion HD Cloning Kit (manufactured by Takara Bio Inc.), and the resulting plasmid was introduced into *E. coli* strain DH5α. The nucleotide sequence on the plasmid extracted from the obtained recombinant strain was confirmed in accordance with routine procedures. The obtained plasmid was designated as "pMW119::PFL."

Example 11

Preparation of mutant *Serratia* microorganism with increased expression of PDHc or PFL and with introduced plasmid expressing enzymes that catalyze reactions A, B, D and E

The plasmids prepared in Reference Examples 7 and 8 were individually introduced into the strains prepared in Reference Example 2 to prepare mutant *Serratia* microorganisms.

SgΔPP/3HA, SgΔG/3HA, and SgΔGPP/3HA were respectively seeded in 5 mL of LB medium containing 25 μg/mL kanamycin and incubated with shaking at 30°C for 1 day. Subsequently, 0.5 mL each of the cultures was seeded in 5 mL of LB medium containing 25 μg/mL kanamycin, and incubated at 30°C for 2 hours with shaking. The cultures were cooled on ice for 20 minutes, and then the bacterial cells were washed with 10%(w/w) glycerol 3 times. The washed pellet was resuspended in 100 μL of 10%(w/w) glycerol, mixed with 1 μL of pMW119::PDHc or pMW119::PFL, and then cooled in an electroporation cuvette on ice for 10 minutes. Electroporation was performed (3 kV, 200 Ω, 25 μF) using

Gene pulser (manufactured by Bio-Rad Laboratories, Inc.), immediately after which 1 mL of SOC culture medium was added and incubated at 30°C for 1 hour with shaking. Subsequently, 50 μL each of the cultures was applied to LB agar medium containing 25 μg/mL kanamycin and 500 μg/mL ampicillin, and incubated at 30°C for 1 day. The resulting strains with introduced pMW119::PDHc were designated as SgΔPP/3HAPDHc, SgΔG/3HAPDHc, and SgΔGPP/3HAPDHc, respectively. The resulting strains with introduced pMW119::PFL were designated as SgΔPP/3HAPFL, SgΔG/3HAPFL, and SgΔGPP/3HAPFL, respectively.

Example 12

Test for production of 3-hydroxyadipic acid and α-hydromuconic acid using mutant *Serratia* microorganism with increased expression of PDHc or PFL and with introduced plasmid expressing enzymes that catalyze reactions A, B, D and E

The mutant *Serratia* microorganisms prepared in Example 11 were cultured in the same manner as in Example 5 except that the culture medium additionally contained 500 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 4.

Reference Example 9

Preparation of mutant *Serratia* microorganism without increased expression of PDHc or PFL and with introduced plasmid expressing enzymes that catalyze reactions A, B, D and E

pMW119 as a negative control was introduced into the strains prepared in Reference Example 3 to prepare mutant *Serratia* microorganisms.

pMW119 was introduced into SgΔPP/3HA, SgΔG/3HA, and SgΔGPP/3HA in the same manner as in Example 11. The resulting strains were designated as SgΔPP/3HApMW, SgΔG/3HApMW, and SgΔGPP/3HApMW, respectively.

Comparative Example 3

Test for production of 3-hydroxyadipic acid and α-hydromuconic acid using mutant *Serratia* microorganism without increased expression of PDHc or PFL and with introduced plasmid expressing enzymes that catalyze reactions A, B, D and E

The mutant *Serratia* microorganisms prepared in Reference Example 9 were cultured in the same manner as in Example 5 except that the culture medium additionally contained 500 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 4.

As seen from Table 4, the mutant *Serratia* microorganisms with increased expression of PDHc or PFL and with an introduced plasmid expressing enzymes that catalyze the reactions A, B, D and E showed improved yields of 3-hydroxyadipic acid and α-hydromuconic acid.

[Table 4]

| | strain | 3 HA yield (%) | HMA yield (%) |
|---|---|---|---|
| Comparative Example 3 | SgΔPP/3HApMW | 6.31 | 0.011 |
| | SgΔG/3HApMW | 2.35 | 0.048 |
| | SgΔGPP/3HApMW | 4.15 | 0.019 |

(continued)

| | strain | 3 HA yield (%) | HMA yield (%) |
|---|---|---|---|
| Example 12 | SgΔPP/3HAPDHc | 7.35 | 0.021 |
| | SgΔG/3HAPDHc | 2.69 | 0.106 |
| | SgΔGPP/3HAPDHc | 7.41 | 0.158 |
| | SgΔPP/3HAPFL | 8.46 | 0.021 |
| | SgΔG/3HAPFL | 3.03 | 0.171 |
| | SgΔGPP/3HAPFL | 6.51 | 0.197 |

Example 13

Preparation of mutant *Escherichia* microorganism with increased expression of PFL and with introduced plasmid expressing enzymes that catalyze reactions A, B, D and E

The plasmids prepared in Reference Examples 7 and 8 were individually introduced into the strains prepared in Reference Example 3 to prepare mutant *Escherichia* microorganisms.

EcΔPP/3HA, EcΔG/3HA, and EcΔGPP/3HA were respectively seeded in 5 mL of LB medium containing 25 μg/mL kanamycin and incubated with shaking at 30°C for 1 day. Subsequently, 0.5 mL each of the cultures was seeded in 5 mL of LB medium containing 25 μg/mL kanamycin, and incubated at 30°C for 2 hours with shaking. The cultures were cooled on ice for 20 minutes, and then the bacterial cells were washed with 10%(w/w) glycerol 3 times. The washed pellet was resuspended in 100 μL of 10%(w/w) glycerol, mixed with 1 μL of pMW119::PFL, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 1 hour with shaking. Subsequently, 50 μL each of the cultures was applied to LB agar medium containing 25 μg/mL kanamycin and 100 μg/mL ampicillin, and incubated at 30°C for 1 day. The resulting strains with introduced pMW119::PFL were designated as EcΔPP/3HAPFL, EcΔG/3HAPFL, and EcΔGPP/3HAPFL, respectively.

Example 14

Test for production of 3-hydroxyadipic acid and α-hydromuconic acid using mutant *Escherichia* microorganism with increased expression of PFL and with introduced plasmid expressing enzymes that catalyze reactions A, B, D and E

The mutant *Escherichia* microorganisms prepared in Example 13 were cultured in the same manner as in Example 5 except that the culture medium additionally contained 100 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 5.

Reference Example 10

Preparation of mutant *Escherichia* microorganism without increased expression of PDHc or PFL and with introduced plasmid expressing enzymes that catalyze reactions A, B, D and E

pMW119 as a negative control was introduced into the strains prepared in Reference Example 3 to prepare mutant *Serratia* microorganisms.

pMW119 was introduced into EcΔPP/3HA, EcΔG/3HA, and EcΔGPP/3HA in the same manner as in Example 13. The resulting strains were designated as EcΔPP/3HApMW, EcAG/3HApMW, and EcΔGPP/3HApMW, respectively.

Comparative Example 4

Test for production of 3-hydroxyadipic acid and α-hydromuconic acid using mutant *Escherichia* microorganism without increased expression of PDHc or PFL and with introduced plasmid expressing enzymes that catalyze reactions A, B, D and E

The mutant *Escherichia* microorganisms prepared in Reference Example 10 were cultured in the same manner as in Example 5 except that the culture medium additionally contained 100 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 5.

As seen from Table 5, the mutant *Escherichia* microorganisms with increased expression of PFL and with an introduced plasmid expressing enzymes that catalyze the reactions A, B, D and E showed improved yields of 3-hydroxyadipic acid and α-hydromuconic acid.

[Table 5]

| | strain | 3 HA yield (%) | HMA yield (%) |
|---|---|---|---|
| Comparative Example 4 | EcΔPP/3HApMW | 6.73 | 0.025 |
| | EcΔG/3HApMW | 4.39 | 0.023 |
| | EcΔGPP/3HApMW | 12.6 | 0.040 |
| | EcΔPP/3HAPFL | 7.62 | 0.038 |
| | EcΔG/3HAPFL | 5.39 | 0.039 |
| | EcΔGPP/3HAPFL | 18.9 | 0.086 |

Reference Example 11

Preparation of plasmid expressing Pck and enzymes that catalyze reactions A, B, D, and E.

To integrate a promoter for constitutive expression of Pck, primers (SEQ ID NOs: 64 and 65) were designed for use in amplification of an upstream 200-b region (SEQ ID NO: 63) of gapA (NCBI Gene ID: NC_000913.3) by PCR using the genomic DNA of *Escherichia coli* K-12 MG1655 as a template, and a PCR reaction was performed in accordance with routine procedures. The resulting fragment, and the fragment obtained by cleaving the pBBR1MCS-2::ATCTOR produced in Reference Example 1 with SacI were ligated together using the In-Fusion HD Cloning Kit, and the resulting plasmid was individually introduced into *E. coli* strain DH5α. The nucleotide sequence on the plasmid isolated from the obtained recombinant strain was confirmed in accordance with routine procedures, and the plasmid was designated as pBBR1MCS-2::ATCTORPgap. Subsequently, to amplify the gene encoding Pck, primers (SEQ ID NOs: 67 and 68) were designed for use in amplification of a continuous sequence comprising the full length of the pck gene (SEQ ID NO: 66) using the genomic DNA of *Serratia grimesii* strain NBRC13537 as a template, and a PCR reaction was performed in accordance with routine procedures. The resulting fragment, and the fragment obtained by cleaving pBBR1MCS-2::ATCTORPgap with SacI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCTORPCK.

Example 15

Preparation of mutant *Escherichia* microorganism with increased expression of Pck and with introduced plasmid expressing enzymes that catalyze reactions A, B, D and E

pMW119::ATCTORPCK or pMW119 as a control was introduced, in the same manner as in Example 13, into EcΔPPR/3HA and EcΔGR/3HA prepared in Example 9. The resulting strains were designated as EcΔPPR/3HAPCK, EcΔGR/3HAPCK, EcΔPPR/3HApMW, and EcΔGR/3HApMW, respectively.

Example 16

Test for production of 3-hydroxyadipic acid and α-hydromuconic acid using mutant *Escherichia* microorganism with increased expression of Pck and with introduced plasmid expressing enzymes that catalyze reactions A, B, D and E

EcΔPPR/3HAPCK, EcΔGR/3HAPCK, EcΔPPR/3HApMW, and EcΔGR/3HApMW prepared in Example 15 were cultured in the same manner as in Example 14. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 6.

As seen from Table 6, the mutant *Escherichia* microorganisms with increased expression of Pck and with an introduced plasmid expressing enzymes that catalyze the reactions A, B, D and E showed further improved yields of 3-hydroxyadipic acid and α-hydromuconic acid.

Table 6]

| | strain | 3 HA yield (%) | HMA yield (%) |
|---|---|---|---|
| Example 16 | EcΔPPR/3HApMW | 4.01 | 0.006 |
| | EcΔGR/3HApMW | 5.48 | 0.016 |
| | EcΔPPR/3HAPCK | 4.41 | 0.007 |
| | EcΔGR/3HAPCK | 6.34 | 0.019 |

Reference Example 12

Preparation of plasmid for expression of Lpd derived from *Escherichia coli* or LpdA derived from *Klebsiella pneumoniae*

The pMW119 expression vector (manufactured by Nippon Gene Co., Ltd.) capable of autonomous replication in *E. coli,* was cleaved with SacI and KpnI to obtain pMW119/SacI, KpnI.

To amplify a gene encoding Lpd derived from *Escherichia coli,* primers (SEQ ID NOs: 69 and 70) were designed for use in amplification of the region comprising the full lengths of lpd (NCBI Gene ID: 944854) and 0.5 kb on its 5' side and 0.1 kb on its 3' side by PCR using the genomic DNA of *Escherichia coli* str. K-12 substr. MG1655 as a template, and a PCR reaction was performed in accordance with routine procedures. The resulting fragment and pMW119/SacI, KpnI were ligated together using the In-Fusion HD Cloning Kit (manufactured by Takara Bio Inc.), and the resulting plasmid was introduced into *E. coli* strain DH5α. The nucleotide sequence on the plasmid extracted from the obtained recombinant strain was confirmed in accordance with routine procedures. The obtained plasmid was designated as "pMW119::EcLPD".

To amplify a gene encoding LpdA derived from *Klebsiella pneumoniae,* gene synthesis was performed for a region comprising the full length of lpdA of *Klebsiella pneumoniae* subsp. pneumoniae MGH78578 (NCBI Gene ID: CP000647, REGION: 142460..143884), and 0.5 kb on the 5' side and 0.5 kb on the 3' side of lpd of *Escherichia coli* str. K-12 substr. MG1655, followed by designing primers (SEQ ID NOs: 69 and 71) for use in amplification of the region by PCR, and then a PCR reaction was performed in accordance with routine procedures. The resulting fragment and pMW119/SacI, KpnI were ligated together using the In-Fusion HD Cloning Kit (manufactured by Takara Bio Inc.), and the resulting plasmid was introduced into *E. coli* strain DH5α. The nucleotide sequence on the plasmid extracted from the obtained recombinant strain was confirmed in accordance with routine procedures. The obtained plasmid was designated as "pMW119::KpLPD."

Example 17

Preparation of mutant *Escherichia* microorganism with increased expression of Lpd and with introduced plasmid for expression of enzymes that catalyze reactions A, B, D and E

The plasmids prepared in Reference Example 12 were individually introduced into the strains prepared in Reference Example 3 to prepare mutant *Escherichia* microorganisms.

pMW119::EcLPD prepared in Reference Example 3 was introduced into EcΔG/3HA, and pMW119::KpLPD prepared in Reference Example 3 was introduced into EcΔPP/3HA, in the same as in Example 13. The resulting strains were designated as EcΔG/3HAEcLPD and EcΔPP/3HAKpLPD, respectively.

Example 18

Test for production of 3-hydroxyadipic acid and α-hydromuconic acid using mutant *Escherichia* microorganism with increased expression of Lpd and with introduced plasmid expressing enzymes that catalyze reactions A, B, D and E

The mutant *Escherichia* microorganisms prepared in Example 17 were cultured in the same manner as in Example 5 except that the culture medium additionally contained 100 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 7.

As seen from Table 7, the mutant *Escherichia* microorganisms with increased expression of Lpd and with an introduced plasmid expressing enzymes that catalyze the reactions A, B, D and E showed improved yields of 3-hydroxyadipic acid and α-hydromuconic acid.

[Table 7]

| | strain | 3 HA yield (%) | HMA yield (%) |
|---|---|---|---|
| Example 18 | EcΔG/3HAEcLPD | 5.17 | 0.041 |
| | EcΔPP/3HAKpLPD | 8.31 | 0.059 |

Reference Example 13

Preparation of plasmid for expression of Lpd with reduced sensitivity to NADH

An amino acid variant of pMW1 19::EcLPD obtained in Reference Example 12 was prepared. Substitution of glutamic acid (E) with lysine (K) at position 354 was performed using Q5 Site-Directed Mutagenesis Kit (manufactured by New England Biolabs, Inc.), pMW119::EcLPD as a template, and oligonucleotides represented by SEQ ID NOs: 72 and 73 as primers. The obtained plasmid was designated as pMW119::EcLPDE354K.

Similarly, substitution of histidine (H) with tyrosine (Y) at position 322 was performed using pMW119::EcLPD as a template, and oligonucleotides represented by SEQ ID NOs: 74 and 75 as primers. The obtained plasmid was designated as pMW119::EcLPDH322Y.

Example 19

Preparation of mutant *Escherichia* microorganism after introduction of Lpd with reduced sensitivity to NADH and with introduced plasmid expressing enzymes that catalyze reactions A, B, D and E

The plasmids prepared in Reference Example 13 were individually introduced into the strains prepared in Reference Example 3 to prepare mutant *Escherichia* microorganisms.

pMW119::EcLPDE354K or pMW119::EcLPDH322Y was individually introduced into EcΔG/3HA prepared in Reference Example 3, in the same manner as in Example 13. The resulting strains were designated as EcΔG/3HAEcLPDE354K and EcΔG/3HAEcLPDH322Y, respectively.

Example 20

Test for production of 3-hydroxyadipic acid and α-hydromuconic acid using mutant *Escherichia* microorganism after introduction of plasmid for expression of Lpd with reduced sensitivity to NADH and enzymes that catalyze reactions A, B, D and E

The mutant *Escherichia* microorganisms prepared in Example 19 were cultured in the same manner as in Example 5 except that the culture medium additionally contained 100 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 8.

As seen from Table 8, the mutant *Escherichia* microorganisms whose sensitivity to NADH was reduced and the plasmid expressing enzymes that catalyze the reactions A, B, D and E showed further improved yield of 3-hydroxyadipic acid and α-hydromuconic acid.

[Table 8]

| | strain | 3 HA yield (%) | HMA yield (%) |
|---|---|---|---|
| Example 20 | EcΔG/3HAEcLPDE354K | 5.83 | 0.046 |
| | EcΔG/3HAEcLPDH322Y | 5.63 | 0.045 |

Example 21

Preparation of mutant *Serratia* microorganism with deletion of one type of pyruvate kinase and increased expression of Lpd, and with introduced plasmid expressing enzymes that catalyze reactions A, B, D, and E

Into SgΔPf prepared in Example 1, were introduced pBBR1MCS-2::ATCTOR in the same manner as in Example 4, and pMW1 19::EcLPD in the same manner as in Example 11. The resulting strain was designated as SgΔPf/3HAEcLPD.

Example 22

Preparation of mutant *Escherichia* microorganism with deletion of one type of pyruvate kinase and increased expression of Lpd, and with introduced plasmid expressing enzymes that catalyze reactions A, B, D, and E

Into EcΔPf prepared in Example 6, were introduced pBBR1MCS-2::ATCTOR in the same manner as in Example 9, and pMW119::EcLPD in the same manner as in Example 17. The resulting strain was designated as EcΔPf/3HAEcLPD.

Example 23

Test for production of 3-hydroxyadipic acid and α-hydromuconic acid using mutant *Serratia* or *Escherichia* microorganism with deletion of one type of pyruvate kinase, and with increased expression of Lpd, and with introduced plasmid expressing enzymes that catalyze reactions A, B, D and E

The mutant *Serratia* or *Escherichia* microorganisms prepared in Examples 21 and 22 were cultured in the same manner as in Example 5 except that the culture medium additionally contained 500 μg/mL or 100 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 9.

As seen from Table 9, even the mutant *Serratia* and *Escherichia* microorganisms with deletion of one type of pyruvate kinase, and with increased expression of Lpd, and with an introduced plasmid expressing enzymes that catalyze the reactions A, B, D and E showed improved yields of 3-hydroxyadipic acid and α-hydromuconic acid.

[Table 9]

| | strain | 3 HA yield (%) | HMA yield (%) |
|---|---|---|---|
| Example 23 | SgΔPf/3HApMW | 2.10 | 0.009 |
| | EcΔPf/3HApMW | 3.97 | 0.023 |
| | SgΔPf/3HAEcLPD | 2.75 | 0.022 |
| | EcΔPf/3HAEcLPD | 4.73 | 0.031 |

Reference Example 14

Preparation of mutant *Escherichia* microorganism with increased expression of only PFL and with introduced plasmid expressing enzymes that catalyze reactions A, B, D and E

pMW119::PFL prepared in Reference Example 8 or pMW119 as a control was introduced into EcWT/3HA prepared in Reference Example 5 in the same manner as in Example 13. The resulting strains were designated as EcWT/3HAPFL and EcWT/3HApMW.

Reference Example 15

Test for production of 3-hydroxyadipic acid and α-hydromuconic acid using mutant *Escherichia* microorganism with increased expression of only PFL and with introduced plasmid expressing enzymes that catalyze reactions A, B, D and E

EcWT/3HAPFL and EcWT/3HApMW prepared in Reference Example 14 were cultured in the same manner as in Example 5 except that the culture medium additionally contained 100 μg/mL ampicillin. The concentrations of 3-hydroxyadipic acid, α-hydromuconic acid, and other products accumulated in the culture supernatant, and of sugars remaining in the culture medium without being used were measured. Additionally, the yields of 3-hydroxyadipic acid and α-hydromuconic acid calculated based on the results according to the Formula (1) are presented in Table 10.

As seen from Table 10, the mutant *Escherichia* microorganisms with increased expression of only PFL and with an introduced plasmid expressing enzymes that catalyze the reactions A, B, D and E showed reduced yields of 3-hydroxyadipic acid and α-hydromuconic acid as compared with the control strain.

[Table 10]

| | strain | 3 HA yield (%) | HMA yield (%) |
|---|---|---|---|
| Reference Example 15 | EcWT/3HApMW | 2.11 | 0.017 |
| | EcWT/3HAPFL | 1.95 | 0.011 |

## Claims

1. A genetically modified microorganism having an ability to produce 3-hydroxyadipic acid and/or α-hydromuconic acid, wherein the reaction to generate acetyl-CoA from pyruvic acid is enhanced and function of pyruvate kinase and/or phosphotransferase system is reduced.

2. The genetically modified microorganism of claim 1, wherein the enhancement of the reaction to generate acetyl-CoA from pyruvic acid is an enhancement of the reaction catalyzed by pyruvate dehydrogenase complex and/or an enhancement of the reaction catalyzed by pyruvate formate-lyase.

3. The genetically modified microorganism of claim 2, wherein the enhancement of the reaction catalyzed by the pyruvate dehydrogenase complex is an enhancement by increased expression of the pyruvate dehydrogenase complex and/or increased activity of the pyruvate dehydrogenase complex.

4. The genetically modified microorganism of claim 3, wherein the increased expression of the pyruvate dehydrogenase complex is achieved by reducing the function of transcriptional repressor of the pyruvate dehydrogenase complex.

5. The genetically modified microorganism of claim 3, wherein the increased activity of the pyruvate dehydrogenase complex is achieved by reducing the sensitivity of the pyruvate dehydrogenase complex to NADH.

6. The genetically modified microorganism of claim 2, wherein the enhancement of the reaction catalyzed by pyruvate formate-lyase is achieved by enhancement by increased expression of pyruvate formate-lyase.

7. The genetically modified microorganism of any one of claims 1 to 6, wherein the reaction that reduces 3-oxoadipyl-CoA to generate 3-hydroxyadipyl-CoA is further enhanced.

8. The genetically modified microorganism of any one of claims 1 to 7, wherein phosphoenolpyruvate carboxykinase reaction is further enhanced.

9. The genetically modified microorganism of any one of claims 1 to 8, wherein the microorganism does not undergo glucose metabolism via the phosphoketolase pathway.

10. A method of producing 3-hydroxyadipic acid and/or α-hydromuconic acid, comprising the step of culturing a genetically modified microorganism of any one of claims 1 to 9.

11. A method of producing a genetically modified microorganism having an ability to produce 3-hydroxyadipic acid

and/or α-hydromuconic acid, comprising a step of enhancing or reducing a function inherent in the microorganism by genetic modification,
wherein the step comprises:

a step (a) of enhancing the reaction to generate acetyl-CoA from pyruvic acid, and
a step (b) of reducing the enzyme function of pyruvate kinase and/or phosphotransferase system.

**12.** The method of claim 11, further comprising a step (c) of enhancing the reaction that reduces 3-oxoadipyl-CoA to generate 3-hydroxyadipyl-CoA.

**13.** The method of claim 11 or 12, further comprising a step (d) of enhancing the phosphoenolpyruvate carboxykinase reaction.

**INTERNATIONAL SEARCH REPORT**

International application No.
**PCT/JP2021/041257**

**A. CLASSIFICATION OF SUBJECT MATTER**

***C12N 1/21***(2006.01)i; ***C12N 15/52***(2006.01)i; ***C12P 7/44***(2006.01)i
FI: C12N1/21 ZNA; C12P7/44; C12N15/52 Z

According to International Patent Classification (IPC) or to both national classification and IPC

**B. FIELDS SEARCHED**

Minimum documentation searched (classification system followed by classification symbols)

C12N1/21; C12N15/52; C12P7/44

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2011-515111 A (GENOMATICA INC.) 19 May 2011 (2011-05-19) claims, abstract, paragraph [0057], example 1 | 1-13 |
| Y | WO 2019/107516 A1 (TORAY INDUSTRIES, INC.) 06 June 2019 (2019-06-06) in particular, claims, abstract | 1-13 |
| Y | WO 2013/163292 A2 (BIOAMBER INC.) 31 October 2013 (2013-10-31) claims, paragraph [0091] | 1-5, 7-13 |
| Y | WO 2014/099725 A1 (GENOMATICA, INC.) 26 June 2014 (2014-06-26) in particular, claims | 1-10 |
| A | WO 2017/033965 A1 (RIKEN) 02 March 2017 (2017-03-02) claims, paragraphs [0038]-[0045], [0103] | 1-13 |
| A | WO 2019/022083 A1 (RIKEN) 31 January 2019 (2019-01-31) paragraph [0110] | 1-13 |
| A | JP 2017-184746 A (MYRIANT CORP.) 12 October 2017 (2017-10-12) claims, paragraphs [0003], [0011], [0045] | 1-13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2022** | **01 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/041257**

**C. DOCUMENTS CONSIDERED TO BE RELEVANT**

| Category* | Citation of document, with indication, where appropriate, of the relevant passages | Relevant to claim No. |
|---|---|---|
| P, Y | WO 2020/230718 A1 (TORAY INDUSTRIES, INC.) 19 November 2020 (2020-11-19) in particular, claims, abstract | 1-13 |
| P, Y | WO 2020/230719 A1 (TORAY INDUSTRIES, INC.) 19 November 2020 (2020-11-19) in particular, claims, abstract | 1-13 |

Form PCT/ISA/210 (second sheet) (January 2015)

**INTERNATIONAL SEARCH REPORT**

International application No.
**PCT/JP2021/041257**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2021/041257**

| Patent document cited in search report | Publication date (day/month/year) | Patent family member(s) | Publication date (day/month/year) |
|---|---|---|---|
| JP 2011-515111 A | 19 May 2011 | JP 2014-147399 A | |
| | | JP 2016-195612 A | |
| | | JP 2018-102317 A | |
| | | JP 2020-174684 A | |
| | | US 2010/0330626 A1 | |
| | | WO 2009/151728 A2 | |
| | | claims, abstract, p. 21, lines 3-15, example 1 | |
| | | US 2009/0305364 A1 | |
| | | US 2011/0195466 A1 | |
| | | US 2012/0115194 A1 | |
| | | US 2012/0264179 A1 | |
| | | US 2012/0309062 A1 | |
| | | US 2013/0095540 A1 | |
| | | US 2015/0167028 A1 | |
| | | US 2017/0130234 A1 | |
| | | US 2020/0248189 A1 | |
| | | EP 3351619 A1 | |
| WO 2019/107516 A1 | 06 June 2019 | US 2020/0291435 A1 | |
| | | in particular, claims, abstract | |
| | | EP 3719121 A1 | |
| WO 2013/163292 A2 | 31 October 2013 | US 2013/0288320 A1 | |
| WO 2014/099725 A1 | 26 June 2014 | US 2015/0329885 A1 | |
| | | US 2014/0329916 A1 | |
| | | US 2019/0300919 A1 | |
| WO 2017/033965 A1 | 02 March 2017 | US 2018/0237813 A1 | |
| | | claims, paragraphs [0064]-[0071], [0116] | |
| | | EP 3342874 A1 | |
| WO 2019/022083 A1 | 31 January 2019 | US 2021/0130854 A1 | |
| | | paragraph [0108] | |
| | | EP 3660156 A1 | |
| JP 2017-184746 A | 12 October 2017 | JP 2015-504688 A | |
| | | US 2015/0044755 A1 | |
| | | claims, paragraph [0003], [0011], [0062] | |
| | | JP 2019-195330 A | |
| | | US 2019/0345437 A1 | |
| | | WO 2013/116244 A1 | |
| | | EP 3312267 A1 | |
| WO 2020/230718 A1 | 19 November 2020 | (Family: none) | |
| WO 2020/230719 A1 | 19 November 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019107516 A
- JP 2013535203 A
- US 20110124911 A1
- JP 2015146810 A
- US 20170298363 A1
- JP 2008527991 A
- US 2020291435 A1
- JP 2015504688 A


### Non-patent literature cited in the description


- *J Biosci Bioeng,* April 2017, vol. 123 (4), 437-443
- **KIM et al.** *J. Bacteriol,* 2008, vol. 190, 3851-3858
- *Proc Natl Acad Sci U.S.A,* 06 June 2000, vol. 97 (12), 6640-6645
- *Biosci Biotechnol Biochem.,* December 2007, vol. 71 (12), 2905-11
- *Journal of Molecular Biology,* 1970, vol. 53, 159
- **NM CALVIN ; PC HANAWALT.** *J. Bacteriol,* 1988, vol. 170, 2796-2801
- **ME KOVACH.** *Gene,* 1995, vol. 166, 175-176
- *Proc Natl Acad Sci USA.,* 06 June 2000, vol. 97 (12), 6640-6645